(19) Europäisches Patentamt
European Patent Office
Office européen des brevets

(11) Publication number: **0 068 378 B1**

(12) **EUROPEAN PATENT SPECIFICATION**

(45) Date of publication of patent specification: **05.03.86**

(21) Application number: **82105411.1**

(22) Date of filing: **21.06.82**

(51) Int. Cl.[4]: **C 07 D 471/04,**
C 07 D 487/04, A 61 K 31/44,
A 61 K 31/50 // (C07D471/04,
235:00, 221:00),(C07D487/04,
241:00, 235:00)

(54) **Novel imidazo(1,2-a)pyridines and pyrazines, processes for their preparation and pharmaceutical compositions containing them.**

(30) Priority: **26.06.81 US 277576**
**08.03.82 US 356052**

(43) Date of publication of application:
**05.01.83 Bulletin 83/01**

(45) Publication of the grant of the patent:
**05.03.86 Bulletin 86/10**

(84) Designated Contracting States:
**AT BE CH DE FR GB IT LI LU NL SE**

(56) References cited:
**EP-A-0 033 094**
**US-A-4 177 274**

**Chemical Abstracts vol. 62, no. 13 21 June 1965 Columbus, Ohio, USA J.P. PAOLINI et al. "Aromaticity in heterocyclic systems. III. The structure and proton magnetic resonance spectra of certain imidazo (1,2-a) pyridines" column 16229b**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Bristol, James Arthur**
**1921 High Hollow Drive**
**Ann Arbor Michigan 48103 (US)**
Inventor: **Puchalski, Chester**
**9 Locust Avenue**
**Dover New Jersey 07801 (US)**
Inventor: **Lovey, Raymond George**
**65 Woodside Avenue**
**West Caldwell New Jersey 07006 (US)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1 (DE)**

**EP 0 068 378 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel imidazo[1,2-a]pyridines and pyrazines, to process for their preparation, to pharmaceutical compositions containing such compounds and to methods for preparing such compositions. The compounds are particularly useful for treating peptic ulcer disease.

Certain substituted imidazo[1,2-a]pyridines and their use as anthelmintic agents are known, e.g. from USP 4,177,274. They differ in structure from the compounds of this invention, and their published use does not suggest any use of structurally related compounds in the treatment of peptic ulcer.

Compounds closely related to, though different in structure from those of this invention, and their use for the treatment of peptic ulcer, have been described in EP—A1—0033094, published after the priority date of this invention.

The imidazo[1,2-a]pyridines and pyrazines of this invention include compounds of the general formula

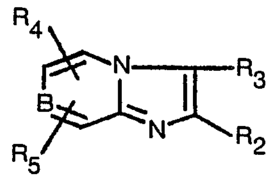

I

wherein B is CH or N; whereby, when B represents CH, then

$R_2$ represents hydrogen, $C_1$—$C_6$alkyl or hydroxy $C_1$—$C_6$alkyl;

$R_5$ represents hydrogen, halogen, or $C_1$—$C_6$alkyl; and either

$R_3$ is $C_1$—$C_6$alkyl, —$CH_2$CN, hydroxy$C_1$—$C_6$alkyl, —NO, —$CH_2$NC or

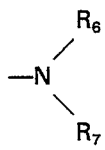

or, provided $R_2$ is not hydrogen, also hydrogen and

$R_4$ being attached to any of positions 5, 6 or 7 of the nucleus, represents any of the groupings —O—$R_8$—Ar, —NH—$R_8$—AR, —$R_8$—Ar, —CH=CH—Ar, —CH=CH—$CH_2$—Ar or —O—$CH_2$—CH=$CH_2$; or

$R_3$ is as defined above and

$R_4$ being attachd to the 8-position of the nucleus, represents any of the groupings:
—CH=CH—Ar, —CH=CH—$CH_2$—Ar or —O—$CH_2$—CH=$CH_2$; or
$R_3$ is —NO, —$CH_2$NC or

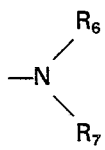

and

$R_4$, being attached to the 8-position of the nucleus, represents —O—$R_8$—Ar, —NH—$R_8$—AR, —$R_8$—Ar; and when B represents N, then

$R_5$ is as above defined,

$R_2$ and $R_3$ are independently selected from hydrogen, $C_1$—$C_6$alkyl, hydroxy$C_1$—$C_6$alkyl, —$CH_2$CN, —NO and —$NR_6R_7$ and

$R_4$ is —O—$R_8$—Ar, —NH—$R_8$—AR, —$R_8$—Ar, —CH=CH—Ar or —CH=CH—$CH_2$—Ar whereby in the above definitions, $R_6$ and $R_7$ are independently selected from hydrogen or $C_1$—$C_6$alkyl;

$R_8$ is a straight- or branched- chain$C_1$—$C_6$alkylene group; and

Ar represents thienyl, furanyl, pyridyl, phenyl or phenyl substituted by one or more substituents selected from halogen and $C_1$—$C_6$alkyl;

the 2,3-dihydro; 5,6,7,8-tetrahydro and 2,3,5,6,7,8-hexahydro derivatives thereof and the pharmaceutically acceptable salts of such compounds.

As used herein, the term "halogen" includes fluoro, chloro, bromo and iodo, with fluoro and chloro being preferred; the term "$C_1$—$C_6$alkyl" means straight and branched chain hydrocarbon groups having up to six carbon atoms such as methyl, ethyl, propyl, butyl, t-butyl, isopropyl, neopentyl, dimethylbutyl etc, whereby methyl and ethyl are preferred. Equally the lower alkylene gorups represented by $R_8$ are such having 1 to 6 carbon atoms with ethylene, methylene and propylene being preferred.

The term "pyridyl" includes the 2-, 3- and 4-isomers and the terms "thienyl" and "furanyl" include the 2- and 3-isomers.

In those instances where $R_5$ is other than hydrogen, such substituent may be at any of the positions 5-, 6-, 7- or 8- of the imidazo[1,2-a]pyridine nucleus or that any of positions 5-, 6- or 8- of the imidazo[1,2-a]pyrazine nucleus which is not occupied by the group $R_4$. The preferred position of the $R_4$-group is the 8-position in the nucleus.

I One preferred group of compounds are those wherein B is CH;

$R_2$ represents —$CH_3$ or $C_2H_5$;

$R_5$ is hdyrogen or —$CH_3$;

$R_3$ is —$NH_2$, —$NHC_2H_5$, —$CH_2CN$ or —$CH_3$ and

$R_4$ being attached to any of positions 5, 6 or 7 is either —O—$R_8$—Ar, —NH—$R_8$—AR, —$R_8$—Ar, —CH=CH—Ar, or —CH=CH—$CH_2$—Ar, with $R_8$ being ethylene, methylene or propylene and Ar being phenyl, o-fluorophenyl, p-fluorophenyl, p-chlorophenyl, thienyl or furanyl.

II Another preferred group of compounds are those wherein B is CH, $R_2$, $R_4$ and $R_5$ are as defined above under I, whereby, however, $R_4$ is attached to the 8-position of the nucleus; and $R_3$ represents —$NH_2$ or —$NHC_2H_5$.

III A further group of interest consists of compounds wherein B is CH and, $R_2$ and $R_5$ are as defined above under group I; $R_3$ is —$CH_2CN$ or —$CH_3$ and $R_4$, being attached to the 8-position of the nucleus, is —CH=CH—Ar or —CH=CH—$CH_2$—Ar, with Ar being as defined under I above.

IV A most preferred group of compounds consists of such having the general formula

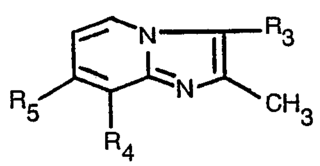

IA

wherein $R_5$ is hydrogen or methyl and either

$R_3$ is —$NH_2$ and

$R_4$ is —$CH_2$—$CH_2$—Ar or —$CH_2$—$CH_2$—$CH_2$—Ar; or

$R_3$ is —$NH_2$, —$CH_2CN$ or —$CH_3$ and

$R_4$ is —CH=CH—Ar or —CH=CH—$CH_2$—Ar whereby Ar represents phenyl or 3-thienyl

V Another most preferred group consists of compounds of formula

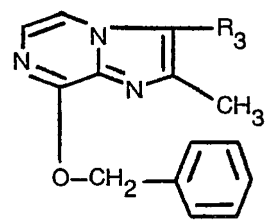

wherein $R_3$ is loweralkyl, —$CH_2CN$, —$NH_2$ or —$NHC_2H_5$.

The compounds of this invention may be obtained by processes generally known for the preparation of compounds having a similar structure. The basic step consists in formation of the (substituted) imidazo[1,2-a]pyridine- or pyrazine-nucleus. Thus, the basic process comprises the following reactions:

A:

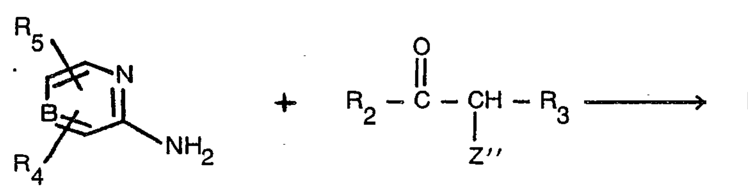

II III

In the above formulae II and III, $R_2$, $R_3$, $R_4$ and $R_5$ are as defined hereinabove, and Z'' represents a good leaving group, preferably a reactive inorganic or organic ester group, such as halogen, tosyl, mesyl etc. Any free amino- or hydroxy groups present in $R_2$ and $R_3$, may advantageously be protected by a protecting group, which is subsequently removed. The reaction is preferably carried out by heating the reactants together (e.g. at reflux temperature) in an inert solvent.

If in the above reaction a compound of Formula III wherein $R_2$ and $R_3$ are both hydrogen and Z'' is chlorine is used (chloracetaldehyde), then $R_2$ and $R_3$ in the final compound are both hydrogen.

These compounds are intermediates useful in the preparation of compounds falling within the scope of this invention, e.g. as used in Step (ii) of process E hereinbelow:

If a compound of Formula III wherein $R_2$ is methyl, $R_3$ is hydrogen and Z'' is chlorine (chloroacetone) is used, then a compound wherein $R_2$ is methyl and $R_3$ is hydrogen or obtained.

For preparing compounds wherein $R_4$ represents $-O-R_8-Ar$, $-O-CH_2CH=CH_2$, $-NH-R_8-Ar$, or $-R_8-Ar$, the following process may be used

B: 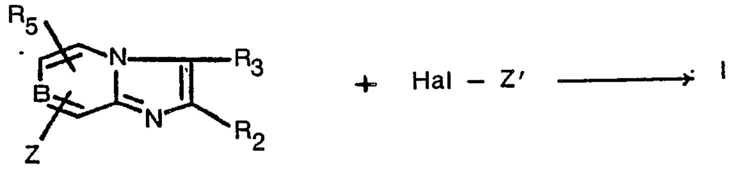


IV V

In the above formula IV and V Hal represents Br, Cl or J; Z represents halogen (Cl, Br, J), OH or $NH_2$, $R_2$, $R_3$ and $R_5$ are as above defined and Z' represents either $-R_8-Ar$ or $-CH_2-CH=CH_2$ with $R_8$ and Ar being as defined above.

The reactants are heated together under standard reaction conditions known from the preparation of similar compounds, e.g. in an inert solvent in the presence of a base. When Z represents halogen, a copper catalyst is preferably used. When Z represents OH, or $NH_2$ the reaction may be carried out with or without such copper catalyst.

The starting compound of Formula IV may be obtained according to process A above, i.e. by reacting a compound of Formula II (wherein $R_4$ is replaced by Z) with a compound of Formula III.

C: Compounds of Formula I wherein $R_4$ represents $-CH=CH-Ar$ or $-CH=CH-CH_2-Ar$ are preferably prepared from a compound of Formula IV wherein Z represents a CHO— group through a Wittig reaction or a modification thereof. Again the starting compounds of Formula IV needed for this process may be prepared according to process A above. The Wittig reagents to be used are preferably well-known dialkylphosphonates. Thus by using a diethylbenzylphosphonate in the above reaction a compound wherein $R_4$ is phenylethenyl is obtained.

D: Alternatively the compounds of this invention wherein $R_4$ represents $-CH=CH-Ar$ may be obtained by reacting a compound of Formula IV wherein Z represents a phosphinylmethyl group [e.g.

$$-CH_2-\overset{\oplus}{P}(OC_2H_5)_2] \quad (P-O^{\ominus})$$

with a compound Ar—CHO. The starting phosphonate of Formula IV may be prepared as exemplified below:

A compound of Formula IV wherein Z represents —CHO (prepared by using process A above) is reduced (e.g. with $NaBH_4$) to the corresponding hydroxymethyl compound.

Chlorination thereof, e.g. with $SOCl_2$, replaces the OH group by Cl and reaction of the resulting compound wherein Z is $-CH_2Cl$ with $P(OC_2H_5)_3$ provides the desired starting compound.

For preparing compounds of Formula I wherein $R_3$ represents the group $CH_2CN$, the following process may be applied:

E: A compound of general Formula I wherein $R_2$, $R_4$ and $R_5$ are as defined for Formula I but wherein $R_3$ is replaced by a group $CH_2X$ (wherein X represents a good leaving group), is reacted with a metal cyanide to yield the desired product. Preferred leaving groups are halogen, alkoxy, aryloxy, mesyl, tosyl, quaternary groups such as $\overset{\oplus}{N}(CH_3)_3.J^{\ominus}$ and quaternary groups wherein the quaternary ion is a non-nucleophilic counter ion such as $BF_4^{\ominus}$, $PF_6^{\ominus}$, $CF_3SO_3^{\ominus}$, $FSO_3^{\ominus}$, etc. Additionally one may carry out the displacement reaction in the presence of a crown ether. The preferred metal cyanides are alkali metal cyanides.

The reaction is carried out under standard conditions, e.g. by heating the reactants in an inert solvent, preferably dimethylformamide. When the quaternary anion is a non-nucleophilic counter ion, the reaction may also be carried out in an aqueous solvent.

The starting compounds of this process may be obtained by standard procedures, e.g. as illustrated below for the quaternary iodide salt:

(i) A compound of Formula II is reacted with a compound of Formula III whereni $R_3$ is hydrogen according to process A above.

(ii) The compound resulting from step (i), which is a compound of Formula I wherein $R_3$ is hdyrogen, is further reacted with dimethylamine hydrochloride and paraformaldehyde by heating the reactants in methanol at reflux temperature.

(iii) The compound of step (ii) is a compound of Formula I wherein $R_3$ represents $-CH_2N(CH_3)_2$ is reacted with $CH_3J$ to yield the desired methiodide.

Starting compounds wherein X represents halogen may be obtained from the corresponding compound wherein X is hydroxy through chlorination, e.g. by treatment with phosphorylchloride.

F: Compounds wherein $R_2$ and/or $R_3$ represent an hydroxy-loweralkyl group may be prepared by reducing a compound of Formula I wherein $R_2$ and/or $R_3$ is the group $-(R')_nCOOR$ (R' being a lower

alkylene group with 1 to 5 carbon atoms, R being a hydrocarbon group and n being zero to one).

Thus, reduction of a compound of Formula I wherein $R_3$ is replaced by $-COOC_2H_5$, e.g. by using lithium aluminium hydride in tetrahydrofuran, yields the corresponding compound of Formula I wherein $R_3$ is $-CH_2OH$.

The starting compound (i.e. a compound wherein $R_2$ and/or $R_3$ is $-(R')_nCOOR$ may be prepared according to process A above by using a compound of Formula III providing the desired carboxylic acid ester group in the 2- or 3- position.

As is obvious to any one skilled in the art, numerous standard reactions may be used for introducing a specific $R_2$ and/or $R_3$ group into the molecule or for substituting one type of $R_2$ and/or $R_3$ by another.

G: Compounds wherein $R_3$ represents —NO may be prepared by nitrousation of a compound of Formula I wherein $R_3$ is hydrogen. The reaction is carried out under standard conditions, e.g. by reaction with a nitrile (soidum nitrite) in the presence of HCl.

H: Compounds of Formula I obtained according to process G above may be used for the preparation of compounds wherein $R_3$ represents $-NH_2$ in that the nitroso compound is subjected to a standard reduction procedure, e.g. by means of zinc powder in acetic acid. Starting compounds having $NO_2$ instead of NO may likewise be reduced.

I: The compounds wherein $R_3$ represents

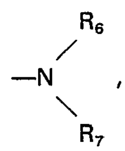
,

with $R_6$ and/or $R_7$ being loweralkyl, are preferably prepared by simple alkylation of compounds of Formula I wherein $R_3$ is an amino group.

J: Compounds of Formula I wherein $R_3$ represents a isocyanomethyl may be prepared by reacting a compound of Formula I wherein $R_3$ is $CH_2NH\overset{O}{C}H$ with $POCl_3$ in the presence of an amine. The starting compound may be obtained by subjecting a compound wherein $R_3$ is $-CH_2OH$ to the following sequence of reaction steps:

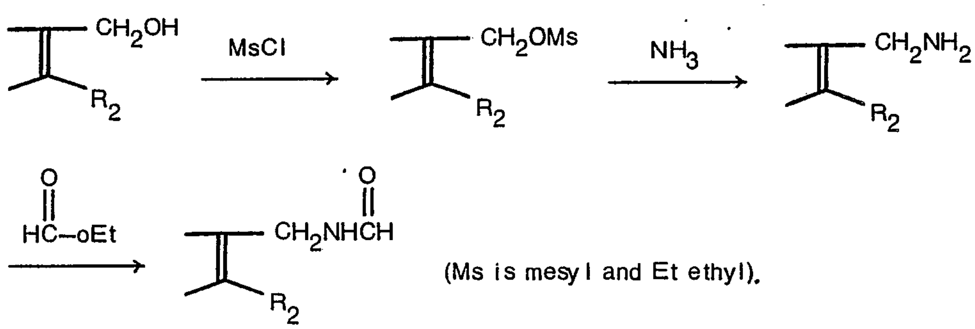


(Ms is mesyl and Et ethyl).

The 2,3-dihydro, 5,6,7,8-tetrahydro and 2,3,5,6,7,8-hexahydro derivatives may be prepared by standard reduction procedures, e.g. by means of $H_2$ in the presence of a palladium catalyst.

It is also obvious to anyone skilled in the art that the sequence of certain reactions may be altered. Thus, for example, one may, in accordance with process (A) above first prepare a compound of the formula

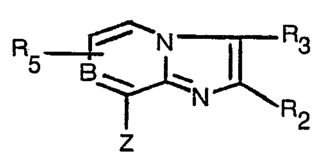

make the above described rearrangements within the groups $R_2$ and $R_3$ and then complete the molecule by carrying out process (B) above.

Example 1

8-Benzyloxy-2-methyl-3-nitroso imidazo[1,2-a]pyridine

*Step A:* Preparation of 2-amino-3-benzyloxypyridine.

In a 12 liter 3-neck round bottom flask equipped with mechanical stirrer and thermometer there were placed 2.5 liters of 40% sodium hydroxide solution, 26.5 g of Adogen 464 (Rg. Trademark) and 2.5 liters of dichloromethane. To this vigorously stirred mixture was added 550 g of 2-amino-3-hydroxypyridine. The temperature was 38°C. The brown orange mixture was cooled to 25°C, and 677.5 g of benzylchloride was added in one portion and the mixture was allowed to separate into 2 phases. The lower aqueous phase was

separated and diluted with 1 liter of ice:water. This solution was then extracted with dichloromethane (3 × 15 liters). The combined dichloromethane extracts were added to the original dichloromethane phase, washed with 1 liter of saturated sodium chloride solution and dried over potassium carbonate. The dichloromethane extract was filtered and concentrated on the rotary evaporator to an orange solid. This solid was dissolved in 1 liter of boiling absolute ethanol, and the solution was filtered. The filtrate was chilled, and the crystals that formed were filtered, washed with 500 ml of ethanol at −10°C, and dried at 50°C. in a vacuum oven, to give the desired product.

*Step B:* Preparation of 8-benzyloxy-2-methyl-imidazo[1,2-a]pyridine

Into a 12 liter 3-necked flask equipped with a mechanical stirrer and condenser, were placed 750 g of 2-amino-3-benzyloxy-pyridine (obtained according to Step A), 6.75 liters of absolute ethanol (one may also use methanol 3 liters) and 360 ml of chloroacetone. The solution was heated under reflux for 4 hours.

An additional 180 ml of chloroacetone was added and the dark solution was heated under reflux for 18 hours. The solvent was evaporated off and the residual dark oil was dissolved in 7 liters of water. The resulting solution was made strongly basic with 15% sodium hydroxide and the basified solution was extracted with several portions (4 × 1.5 liters) of dichloromethane. The extracts were combined and washed with brine and the washed extracts evaporated down to a dark gum which was boiled with 7 liters of diisopropyl ether. The solution was decanted from insoluble material through a glass wool plug, and the filtrate was chilled. The resulting crystals were filtered and washed with cold diisopropyl ether.

Recrystallization provided the pure product of this example (m.p. 93—95°C.)

*Step C:* Preparation of title compound

To a stirred mixture of 10.0 g (42.2 mmol) of 8-benzyloxy-2-methyl-imidazo[1,2-a]pyridine, 150 ml of water and 150 ml of chloroform is added cautiously (EXOTHERM) 179.5 ml (2.15 moles) of concentrated hydrochloric acid and the resultant mixture is heated to an internal temperature of approximately 55°C. To this stirred and heated mixture is added at a rate of approximately 7 ml/minute a solution of 151 g (2.11 moles) sodium nitrate (97%) in 600 ml of water to produce a vigorous, but manageable, reflux. When the addition is complete, the reaction mixture is allowed to cool to room temperature. The lower ($CHCl_3$) layer is drawn off and the aqueous layer extracted with three — 150 ml portions of chloroform. The combined chloroform extracts are washed with two — 450 ml volumes of 2.4 *M* sodium carbonate solution, then with a single 500 ml portion of saturated aqueous sodium chloride. The extracts are concentrated *in vacuo* (rotary evaporator, 45°C.) to less than one-half the original volume, dried over anhydrous sodium sulfate and evaporated to a viscous oil. Chromatography of the oil on silica gel, eluting with chloroform/ethyl acetate (1/1) yields the title compound as a given crystalline solid, mp 147.5—149.5°C. (dec).

Example II

3-Amino-8-benzyloxy-2-methyl-imidazo[1,2-a]pyridine phosphate

A.

To a stirred mixture of 3.2 g (12 mmol) of 8-benzyloxy-2-methyl-3-nitroso-imidazo[1,2-a]pyridine in 24 ml of glacial acetic acid and 33.5 ml of water is added portionwise 3.23 g (49.5 mmol) of zinc powder over a two hour period. When addition is complete, the reaction mixture is stirred at room temperature for 30 minutes. The mixture is filtered through Celite, and the filtrate diluted with 75 ml each of ether and methylene chloride and washed with a solution of 250 ml of 1.7 *M* sodium hydroxide. The resultant emulsion is filtered through Celite and the Celite pad washed thoroughly with 250 ml of hot chloroform.

The layers of the filtrate are separated and the aqueous phase extracted with the chloroform used to wash the Celite pad. The combined organic extracts are washed with two-125 ml portions of water and one-150 ml volume of saturated aqueous sodium chloride and then concentrated under reduced pressure to a volume of approximately 100 ml. The concentrate is dried over anhydrous sodium sulfate and the solvent removed under reduced pressure (rotary evaporator, 40°C.) to give a slightly tacky brown powder that is triturated in ether (75 ml) — methylene chloride (1 ml) to yield the free base of the title compound as a light brown powder, mp 126—131.5°C. (dec).

B.

850 mg (3.37 mmol) of the free base is dissolved in approximately 40 ml of dry acetonitrile. To the stirred solution is added 8 ml of an acetronitrile solution containing 3.4 mmol of phosphoric acid. A precipitiate forms and the mixture is diluted with 70 ml of ether and filtered. The solid is triturated in 60 ml of fresh ether and filtered to give the crude phosphate salt which upon recrystallization from methanol — ethyl acetate yields the title phosphate salt containing 1.3 moles of water of crystallization, mp 214—214.5°C. (dec.).

Example III

5-Benzyloxy-2,3-dimethylimidazo[1,2-a]pyridine

Sodium hydride (50% in oil, 4.8 g) is added to a solution of benzyl alcohol (6.48 g) in dimethylformamide (200 ml). 5-chloro-2,3-dimethylimidazo[1,2-a]pyridine hydrobromide (10.5 g) is added and the mixture is stirred at room temperature for two hours.

The solvent is removed *in vacuo* and the residue is partitioned between water (500 ml) and ether (400 ml). The ether layer is separated, dried over anhydrous sodium sulfate and concentrated *in vacuo* to yield a yellow oil which is chromatographed on silica gel (300 g) using ether as an eluant. After an initial fore-run of a yellow oil, the product is obtained which crystallizes from 1-chloro-butane-hexane mixture.

Similarly, the use of benzylamine in place of benzyl alcohol gives 5-benzylamino-2,3-dimethyl-imidazo[1,2-a]pyridine.

Example IV

7-Benzyloxy-3-cyanomethyl-2-methylimidazo[1,2-a]pyridine

*Step A:* Preparation of 3-chloro-4-oxopentanonitrile.

Into a 1-neck, 3-1 round bottom flask there were placed 1 l diethylether ($Et_2O$) and 100 g 4-oxopentanonitrile. The magnetically stirred solution was cooled to 0—5°C., one drop $HCl/Et_2O$ added, and 185 ml 97% of $SO_2Cl_2$ previously chilled to 5—10°C. was added all at once. The ice bath was removed, and the pale greenish-yellow solution was warmed to 20 ± 1°C. over 5 min. by a hot water bath. The temperature was maintained at 20 ± 1°C. by a cold water bath for 2½ hours. The pale yellow solution was evaporated on a rotary evaporator in a 30°C. water bath at 80 mm vacuum, and carefully watched. Near the end of solvent removal, the *instant* the near-colourless residue began to turn orange, the flask was removed *quickly* and diluted with 1 l cold $Et_2O$. One ml $SO_2Cl_2$ was added and stirred 15 min., and the orangish solution was diluted with 1 l cold $Et_2O$.

The ether solution was washed with 1 l cold was washed with 1 l cold saturated $NaHC_3$-solution which was in turn extracted with 2 × 1/2 l cold $CH_2Cl_2$. The $CH_2Cl_2$ was evaporated, the residue dissolved in 200 ml $Et_2O$, and added to the bicarbonate-washed ether solution. The ether was extracted by 2 × 1 l col 10% $NaHSO_3$-solution and discarded. The bisulfite solution was cooled in an ice bath and 25% NaOH was added slowly to attain pH 7 (ca. 100 ml), 100 g $NaHCO_3$ was added to saturate the solution, and it was extracted with 5 × 1 l $CH_2Cl_2$; 25 g $K_2CO_3$ was added followed by extraction with 1 l $CH_2Cl$, and this was repeated with another 25 g $K_2CO_3$ — 1 l $CH_2Cl_2$. The combined extracts were dried over $MgSO_4$, and evaporated to leave a brown-orange oil, estimated from pmr to contain 3-Cl isomer, 5-Cl isomer, some unknown compounds and $CH_2Cl_2$.

Distillation of this oil through a jacketed 15-cm Vigreux column at 0.3 mmHg gave 3-chloro-4-oxopentanonitrile (b.p. 83—93°C).

*Step B:* Preparation of title compound.

A solution of 2-amino-4-phenylmethoxypyridine (1.45 g) triethylamine (0.73 g) and 3-chloro-4-oxopentanonitrile (0.95 g) in methanol (40 ml) is heated under reflux for three hours. After one hour, additional 3-chloro-4-oxopentanonitrile (0.5 g) and triethylamine (0.36 g) is added. The solvent is removed *in vacuo*, and the residue is partitioned between methylene chloride (50 ml) and 2% sodium hydroxide (50 ml). The methylene chloride layer is removed and concentrated *in vacuo* to give 1.5 g of crude product

A solution of crude product (1.l g) and 0.6 ml pyridine in 20 ml p-dioxane is cooled in an ice bath and treated with 0.6 ml trifluoroacetic anhydride in 2 ml p-dioxane. After removal of the ice bath, the reaction is stirred for one hour, diluted with water (300 ml) and treated with saturated sodium bicarbonate to pH 7—8. Filtration gives 0.8 g of a solid which is chromatographed on silica gel (125 g) using 3% methanol in methylene chloride to give the product which crystallizes from ethyl acetate.

Similarly, the use of 2-amino-4-benzylaminopyridine in the above procedure yields 7-benzylamino-3-cyanomethyl-2-methyl-imidazo[1,2-a]pyridine.

Example V

8-Alloyloxy-3-cyanomethyl-2-methyl-imidazo[1,2-a]pyridine

A.

8-Benzyloxy-3-cyanomethyl-2-methyl-imidazo[1,2-a]pyridine (40 g, 0.14 mol), 1,4-cyclohexadiene (50 g, 0.62 mol), dimethylformamide (600 ml) and palladium black (2 g) are stirred together and heated. At 45°C., sudden exotherm occurs and the temperature rises rapidly to 75—80°C. Heating is discontinued and the mixture stirred for 1 hour. The catalyst is removed by filtration and the cyclohexadine removed *in vacuo*. The dimethylformamide is removed *in vacuo* (0.1 mm) at 55°C. to afford 3-cyanomethyl-2-methyl-8-hydroxy-imidazo[1,2-a]pyridine (27 g, 0.14 mol).

B.

3-3-cyanomethyl-2-methyl-8-hydroxy-imidazo[1,2-a]pyridine (60.5 g, 0.32 mol) and dry dimethylformamide (1600 ml) are stirred together and sodium hydride (15.4 g, 0.32 mol, 50% in oil) added during 15 minutes. The mixture is stirred 1.5 hours after the addition of sodium hydride. Allyl bromide (38.7 g, 0.32 mol) is added dropwise during 1 hour. After the addition of allyl bromide, the reaction is stirred for an additional 1 hour. The dimethylformamide is removed *in vacuo*. The residue partitioned between water (1 liter) and chloroform (3 liters), the organic layer separated, washed with water (1 liter) dried over anhydrous magnesium sulfate and the solvent removed *in vacuo*. Residual dimethylformamide is removed *in vacuo* (0.1 mm). The residual oil is dissolved in chloroform (300 ml) and filtered through a silica gel plug (100 g, tlc grade 60H) to remove coloured materials. This process yields 29.2 g of an oil.

C.

Treatment of the oil produced in Step B with ethereal hydrogen chloride gives 8-allyloxy-3-cyanomethyl-2-methylimidazo[1,2-a]pyridine (29.9 g, 0.11 mol.) mp 177—179°C.

Example VI

3-Cyanomethyl-2-methyl-7-(2-phenylethyl)-imidazo[1,2-a]pyridine

*Step A:* Preparation of 3-Dimethylaminomethyl-2-methyl-7-(2-phenylelthyl)-imidazo[1,2-a]pyridine.

Into a one-liter flask there was placed 2-methyl-7-(2-phenylethyl)-imidazo[1,2-a]pyridine (112 g) dimethylamine hydrochloride, paraformaldehyde (15.23 g) and methanol (450 ml) and the mixture was refluxed with stirring for 1.5 hours. Thereafter the mixture was boiled, open to the air, for 3/4 hours. After cooling to room temperature and treatment with concentrated hydrochloric acid (45 ml) the mixture was stirred for 18 hours, filtered and the thick white solid mass formed was washed with methanol and 200 ml of anhydrous ether and dried.

*Step B:* Preparation of 3-dimethylaminomethyl-2-methyl-7-(2-phenylelthyl)-imidazo[1,2-a]pyridine methiodide.

3-Dimethylaminomethyl-2-methyl-7-(2-phenylelthyl)-imidazo[1,2-a]pyridine hydrochloride (1.325 g) was dissolved in 4.5 liters of hot water. The solution was made strongly basic with 50% NaOH-solution. The chilled mixture was extracted with dichloromethane (3 × 1.5 liters) and the combined extracts were washed with brine (1.5 liters). The dichloromethane extract was concentrated on a rotary evaporator. The residual oil was dissolved in 2.5 liters of ethanol. With stirring the solution was cooled and methyl iodide (232 ml) was added dropwise over a period of 1.5 hours. The mixture was allowed to warm to room temperature overnight under continued stirring. The white precipitate was collected after about 18 hours of stirring, washed with 1.5 liters of ethanol and 3 liters of ether. The resulting product is ready for use in Step C below.

*Step C:* Preparation of title compound.

A mixture of the methiodide of Step A (1,552 g) and sodium cyanide (310 g) in 8.4 liters of dimethylformamide was stirred and heated in a steam bath for one hour.

The dark brown reaction mixture was poured into 30 liters of ice water and the mixture was stirred for one hour. The brown solid product was collected, washed with cold water and allowed to air dry. This material was dissolved in 3.8 liters of hot methanol and treated with hydrogen chloride gas until strongly acidic. The mixture was cooled and the product collected. After washing with methanol, acetonitrile and finally with ether the title product as the hydrochloride salt was obtained.

The salt was re-suspended in water and made strongly alkaline with 10% sodium hydroxide. The product was extracted with dichloromethane (3 × 2.5 liters) and the combined extracts concentrated on a rotary evaporator. The residue was dissolved in 3.6 liters of hot acetonitrile, and the resulting solution filtered through a glass wool plug and the filtrate was refrigerated overnight. The desired product was then washed with cold acetonitrile (mp 118°C.).

Example VII

3-Amino-2-methyl-8-(*trans*-2-phenylethenyl)-imidazo[1,2-a]pyridine hydrochloride

A. 2-Methyl-8-Formylimidazo[1,2-a]pyridine

A mixture of 2-aminonicotinaldehyde (92.8 g, 0.76 mol) and bromoacetone (114.5 g, 0.84 mol) in dimethoxyethane (980 ml) is stirred for 2 hours at room temperature and then heated at 65° with stirring for 14 hours. The solid which separates is isolated by filtration, dissolved in 800 ml absolute ethanol and heated under reflux for 6 hours. The ethanol solvent is removed under reduced pressure and the residuce treated with 138 ml 6*N* hydrochloric acid in 750 ml water for 0.5 hour. The acidic aqueous layer is washed with ether (2 × 300 ml) and basified with cooling (78 ml 50% sodium hydroxide and 25 g sodium bicarbonate). The aqueous layer is extracted with dichloromethane. The extracts are combined and dried over anhydrous sodium sulfate. Following filtration, the solvent is removed under reduced pressure to afford 2-methyl-8-formyl-imidazo[1,2-a]pyridine, mp 136—139.5°C.

B. 2-Methyl-8-hydroxymethylimidazo[1,2-a]pyridine

To a stirred suspension of 56.8 g (0.36 mol) and 2-methyl-8-formylimidazo[1,2-a]pyridine in 400 ml isopropanol at 0° is added in portions 8 g (0.21 mol) sodium borohydride. The reaction mixture is stirred at room temperature for an additional 2 hours. The excess sodium borohydride is decomposed by the addition of distilled water and the solution concentrated under reduced pressure at 50°C. The residue is dissolved in water and extracted with chloroform. The chloroform extracts are combined and dried over anhydrous sodium sulfate. Following filtration, the chloroform is removed under reduced pressure to give 2-methyl-8-hydroxymethylimidazo[1,2-a]pyridine.

C. 2-Methyl-8-chloromethylimidazo[1,2-a]pyridine

2-Methyl-8-hydroxymethylimidazo[1,2-a]pyridine 21.4 g (0.13 mol) is dissolved in 400 ml dichloromethane. To the solution at 0°C. is added dropwise with stirring 19 ml of thionyl chloride. The

reaction mixture is stirred for one hour and the dichloromethane is removed under reduced pressure. The residue is dissolved in distilled water, neutralized at 0°C. with ammonium hydroxide and extracted with dichloromethane. The extracts are combined and dried over anhydrous sodium sulfate. Following filtration, the dichloromethane is removed under reduced pressure to give 2-methyl-8-chloromethylimidazo[1,2-a]pyridine, mp. 110—112°C.

D. Diethyl (2-methyl-8-imidazo[1,2-a]pyriylmethyl) phosphonate

2-Methyl-8-chloromethylimidazo[1,2-a]pyridine 37.7 g (0.21 mol) and 91 ml triethylphosphite are heated together at 145—150°C for 2 hours. Upon cooling, the residue is triturated with petroleum ether and dissolved in ether. Insolubles are removed by filtration and the ether is evaporated under reduced pressure. The oil obtained is dissolved in dichloromethane and dried over anhydrous sodium sulfate. Following filtration, the dichloromethane is removed under reduced pressure to give diethyl (2-methyl-8-imidazo[1,2-a]pyridylmethyl) phosphonate as an oil.

E. 2-methyl-8-(2-phenylethenyl)-imidazo[1,2-a]pyridine

A solution of 48.5 g (0.17 mol) diethyl (2-methyl-8-imidazo[1,2-a]pyridylmethyl) phosphonate and 19.4 ml benzaldehyde in 400 ml dimethoxyethane is added dropwise to a stirred suspension of sodium hydride (11.6 g, 0.48 mol) in dimethoxyethane at 0°C.

After stirring overnight, the dimethoxyethane is removed under reduced pressure. The residue is dissolved in water and extracted with dichloromethane. The dichloromethane extracts are combined and dried over anhydrous sodium sulfate. Following filtration, the dichloromethane is removed under reduced pressure. Re-crystallization from ethyl acetate gives 2-methyl-8-(2-phenylethnylimidazo[1,2-a]pyridine, mp 101—105°C.

F. 3-Nitroso-2-methyl-8-(2-phenylethenyl)imidazo[1,2-a]pyridine

To a solution of 5.0 g (0.02 mol) 2-methyl-8-(2-phenylethenyl)-imidazo[1,2-a]pyridine dissolved in 40 ml acetic acid and 100 ml water at 5°C. is added in portions over 10 minutes, 2.7 g (0.04 mol) sodium nitrate. The mixture is stirred at 0°C. for 20 minutes and at room temperature for 2 hours. Additional water (50 ml) is added, the solid is isolated by filtration and washed thoroughly with distilled water (4 × 500 ml).

Re-crystallization from ethyl acetate gives 3-nitroso-2-methyl-8-(2-phenylethenyl)-imidazo[1,2-a]pyridine, mp 158—160°C.

G. 3-Amino-2-methyl-8-(2-phenylethenyl)-imidazo[1,2-a]pyridine

To a stirred mixture of 3.0 g (0.01 mol) 3-nitroso-2-methyl-8-(2-phenylethenyl)-imidazo[1,2-a]pyridine in 50 ml of glacial acetic acid and 50 ml of water at 0°C. is added in portions 3.0 g (0.046 mol) zinc. When the addition is complete the mixture is stirred at 0°C for 1 hour. The mixture is filtered through celite; the filtrate is diluted with water and dichloromethane and basified at 10°C. with 80 ml of 5*N* sodium hydroxide. The resultant emulsion is filtered through a pad of celite and the celite pad is washed thoroughly with hot chloroform. The layers of the filtrate are separated and the aqueous phase is extracted with chloroform. The organic layer is washed with water and brine and dried over anhydrous sodium sulfate.

Following filtration, the chloroform is removed under reduced pressure to give 3-amino-2-methyl-8-(phenylethenyl)imidazo[1,2-a]pyridine. 1.0 g (0.004 mol) of the free base is dissolved in ethyl acetate and treated with 2 ml of 3.4M etheneal hydrogen chloride. Re-crystallization of the solid from methanol/ethyl acetate gives, 3-amino-2-methyl-8-(*trans*-2-phenylethenyl)-imidazo[1,2-a]pyridine hydrochloride, mp 241—250°C (dec.).

Example VIII

2-Methyl-3-isocyanomethyl-8-phenylmethoxy-imidazo[1,2-a]pyridine

2-Methyl-3-formylaminomethyl-8-phenylmethoxy-imidazo[1,2-a]pyridine 100 mg (0.29 mmol) is added to 6 ml dichloromethane containing 0.6 ml diisopropyl ethylamine and 0.1 ml phosphorous oxychloride. The mixture is stirred for 2 hours and diluted with water. The dichloromethane layer is separated, washed with saturated sodium bicarbonate, brine and dried over anhydrous magnesium sulfate.

Following filtration the dichloromethane is removed under reduced pressure to give 2-methyl-3-isocyanomethyl-8-phenylmethoxyimidazo[1,2-a]pyridine, m.p.

Example IX

*trans*-2,3-Dimethyl-8-(2-phenylethenyl)-imidazo[1,2-a]pyridine hydrochloride.

A. 2,3-Dimethyl-8-formylimidazo[1,2-a]pyridine

A solution of 207 g (1.7 mol) 2-aminonicotinaldehyde and 300 g (2.0 mol) 3-bromo-2-butanone in 150 ml dichloromethane is heated on a steam bath allowing the solvent to distill and the mixture is maintained at 100—105°C for 2 hours.

The reaction mixture is dissolved in dilute hydrochloride acid and extracted with ether. The aqueous layer is neutralized with 20% sodium hydroxide. The solid precipitate is isolated by filtration and recrystallized from ethyl acetate to give 2,3-dimethyl-8-formylimidazo[1,2-a]pyridine, mp 145—148°C.

B. *trans*-2,3-dimethyl-8-(2-phenylethenyl)imidazo[1,2-a]pyridine hydrochloride

A stirred solution of 116 g (0.51 mol) diethylbenzylphosphonate in 300 ml dimethylformamide is treated with 28 g (0.052 mol) sodium methoxide.

2,3-Dimethyl-8-formylimidazo[1,2-a]pyridine (80 g, 0.46 mol) is added in portions over 35 minutes while maintaining the temperature between 30—35°C. After stirring at room temperature 2.5 hours, the solvent is removed under reduced pressure and the residue portioned between 300 ml dichloromethane and 500 ml water.

The dichloromethane layer is separated and the solvent is removed under reduced pressure. The solid is triturated with ether (3 × 100 ml) and insolubles are removed from the ether solutions by filtration. The ether filtrates are combined and treated with ethereal hydrogen chloride. Re-crystallization of the solid from water gives *trans*-2,3-dimehyl-8-(2-phenylethenyl)-imidazo[1,2-a]pyridine, mp 240—255°C.

Example X

8-Benzyloxy-2,3-dimethylimidazo[1,2-a]pyridine

A. Mixture of 8-bromo- and 8-chloro-2,3-dimethylimidazo[1,2-a]pyridine

A stirred mixture of 6.38 g of 2-amino-3-chloro-pyrazine, 7.44 g of 3-bromo-2-butanone and 2.5 ml of anhydrous methanol in a bath maintained at 100° was heated for 18 hours. The mixture was cooled to room temperature and partitioned between aqueous sodium bicarbonate and methylene chloride. The layers were separated and the aqueous phase extracted with methylene chloride. The organic extracts were combined and dried over anhydrous sodium sulfate. The solvent was removed at reduced pressure and the residue was chromatographed on silica gel. A mixture of 8-chloro- and 8-bromo-2,3-dimethyl-imidazo[1,2-a]pyridine, m.p. 169.5°—172°C., was isolated upon crystallization of the residue from ethyl acetate.

B.

A solution of 3.13 g benzyl alcohol in 10 ml of dry dimethylformamide (DMF) was added to a stirred suspension of 1.39 g of 50% sodium hydride-mineral oil in 20 ml dry DMF and stirred at room temperature for two hours. A cooled (about 15°C) solution of 4.79 g of the product of Step A in 50 ml dry DMF was added and the reaction mixture was stirred at room temperature for 16 hours. The reaction mixture was concentrated on a rotary evaporator under reduced pressure (55°C/0.2 torr.), and the residue was partitioned between water and methylene chloride.

Example XI

8-Benzyloxy-2-methyl-imidazo[1,2-a]pyrazine

A. 8-Chloro-2-methyl-imidazo[1,2-a]pyrazine

A mixture of 0.53 g of 2-amino-3-chloropyrazine and 0.84 g of 90% chloroacetone was heated at 100°C. for three hours. Then 0.41 g of triethylamine was added and the heating was continued for another 17 hours. Methylene chloride and aqueous sodium bicarbonate were added to the reaction mixture which was stirred vigorously. The organic and aqueous layers were separated and the aqueous phase extracted with methylene chloride. The organic extracts were combined, washed with water and dried over anhydrous magnesium sulfate. The solvent was removed under reduced pressure, and the residual tar was stirred with 1:1 hexaneether. The insolubles were removed and the solvent evaporated under reduced pressure, yielding 8-chloro-2-methyl-imidazo[1,2-a]pyrazine, m.p. 127.5°—130°C.

B.

A solution of 1.84 g of benzyl alcohol in 10 mg dry DMF was added to a stirred suspension of 0.86 g of 50% sodium hydride-in-mineral oil in 5 ml of dry DMF and the mixtures stirred at room temperature for 30 minutes.

A cooled (ca. 10°—15°C.) solution of 2.60 g of 8-chloro-2-methyl-imidazo[1,2-a]pyrazine in 15 ml of dry DMF was added to the mixture, and the reaction mixture was stirred at room temperature for 16 hours. The reaction mixture was concentrated under reduced pressure and the residue triturated with ether and filtered to obtain 8-benzyloxy-2-methyl-imidazo[1,2-a]pyrazine, as determined by spectroscopic and combustion analysis, m.p. 99.5°—101.5°C.

Example XII

8-Benzyloxy-2-methyl-3-nitroso-imidazo[1,2-a]pyrazine

A solution of 14.2 g 2-methyl-8-phenylmethoxy-imidazo[1,2-a]pyrazine, 129.3 g n-butyl nitrite and 142 ml p-dioxane was heated under reflux for 0.5 hour and decanted from a small amount of gum. The supernatant solution was stirred under vacuum (40°/0.1 mm) and the residue azeotroped with cyclohexane to give 8-benzyloxy-2-methyl-3-nitroso-imidazo[1,2-a]pyrazine as a soft, green solid which was identified by pmr and ms.

Example XIII

3-Amino-8-benzyloxy-2-methyl-imidazo[1,2-a]pyrazine

8-Benzyloxy-2-methyl-3-nitroso-imidazo[1,2-a]pyrazine (18.3 g) was dissolved in 280 ml acetic acid and diluted using 140 ml water and the solution cooled to 18°C.; additional acetic acid (100 ml) was then added. Zinc powder (19.2 g) was added in portions over 10 minutes at 18—30°C. and then stirred 2 hours at room temperature.

The reaction mixture was concentrated *in vacuo* at 45°C. and the residue dissolved in 700 ml of a mixture of dichloromethane/water (5/2, V/V) and basified with 100 ml 2.5*M* sodium hydroxide. The resultant suspension was filtered through a celite pad, the filter cake washed with dichloromethane and the combined filtrate and washings separated. The aqueous layer was extracted with dichloromethane (3 × 100 ml) and the extracts were combined and dried over anhydrous sodium sulfate. The solvent was removed *in vacuo* to leave a brown solid. Flash chromatography on silica gel using ethyl acetate gave 3-amino-8-benzyloxy-2-methyl-imidazo[1,2-a]pyrazine, m.p. 126.5—133°C.

Recrystallization from ethyl acetate gave an analytical sample, m.p. 134.5—136°C. Treatment of the free base with etheral hydrogen chloride gave 3-Amino-8-benzyloxy-2-methyl-imidazo[1,2-a]pyrazine hydrochloride, m.p. 119.5—120.5°C. (dec).

According to the processes outlined above, using the appropriate starting compounds, the following compounds may be obtained.

1. 2,3-Dimethyl-8-[1-E-(3-phenylpropenyl)]-imidazo[1,2-a]pyridine; (m.p. of hydrochloride salt: 201—204°C., dec.).
2. 2,3-Dimethyl-8-[(2-phenyl)-ethenyl]-imidazo[1,2-a]pyridine; (m.p. of hydrochloride methanolate ($\cdot HCl \cdot CH_3OH$): 243—255°C.)
3. 3-Cyanomethyl-2-methyl-8-[E-(2-phenyl-1-ethenyl)]-imidazo[1,2-a]pyridine; m.p. 133—136°C.
4. 3-Cyanomethyl-2-methyl-8-[E-1-(3-phenyl-propen-1-yl)]-imidazo[1,2-a]pyridine; m.p. 143—145.5°C.
5. 3-Cyanomethyl-2-methyl-6-(2-phenylethyl)-imidazo[1,2-a]pyridine; m.p. 139—141°C.
6. 3-Amino-8-benzyloxy-2-ethyl-imidazo[1,2-a]pyridine; m.p. 109—110°C.
7. 3-Amino-8-benzyloxy-2,7-dimethyl-imidazo[1,2-a]pyridine; m.p. of the hydrochloride monohydrate: 181—183°C.
8. 3-Amino-2-methyl-8-(2-phenylethyl)-imidazo[1,2-a]pyridine; m.p. of the hydrochloride including 1 mole methanol and 1/4 mol. $H_2O$: 74.5°C.
9. 3-Amino-2-methyl-8-(3-thienylmethoxy)-imidazo[1,2-a]pyridine; m.p. of hydrochloride: 187°C. (dec.)
10. 3-Dimethylamino-2-methyl-8-phenylmethoxy-imidazo[1,2-a]pyridine; m.p. of hydrochloride: 187°C. (dec.)
11. 3-Amino-8-benzyloxy-2,6-dimethyl-imidazo[1,2-a]pyridine; m.p. 141—142°C.
12. 8-Benzyloxy-3-ethylamino-2-methyl-imidazo[1,2-a]pyridine; m.p. of hydrochloride: 191°C. (dec.)
13. 3-Amino-2-methyl-8-[(3-thienylmethyl)-amino]-imidazo[1,2-a]pyridine; m.p. 161—163°C. (dec.)
14. 3-Amino-2-methyl-8-[Z-(2-phenylethenyl)]-imidazo[1,2-a]pyridine; m.p. of the 1/3 hydrate: 116—125°C.
15. 3-Amino-8-(4-chlorophenylmethoxy)-2-methyl-imidazo[1,2-a]pyridine; m.p. of the 1/4 hydrate: 155—158°C. (dec.)
16. 3-Amino-2-methyl-7-(2-phenylethyl)-imidazo[1,2-a]pyridine; m.p. 143—144°C.
17. 3-Amino-2-methyl-8-(2-thienylmethoxy)-imidazo[1,2-a]pyridine; m.p. of 2/3-hydrate: 147—149°C.
18. Trans-3-Amino-2,6-dimethyl-8-(2-phenylethenyl)-imidazo[1,2-a]pyridine; m.p. of hydrochloride: 273—275°C.
19. 3-Amino-8-(2-fluorophenylmethoxy)-2-methyl-imidazo[1,2-a]pyridine; m.p. of the 1/4 hydrate: 151—152.5°C.
20. 3-Amino-8-(4-fluorophenylmethoxy)-2-methyl-imidazo[1,2-a]pyridine; m.p. of 1/4 hydrate: 165—166°C.
21. 3-Amino-8-benzylamino-2-methyl-imidazo[1,2-a]pyridine; m.p. 145—155°C. (dec.)
22. 3-Amino-2-methyl-8-(2-phenylethenyl)-imidazo[1,2-a]pyridine; m.p. of hydrochloride with 2/3 mol. $H_2O$: Decomposition at 241—250°C.
23. 3-Amino-2-methyl-6-(2-phenylethyl)-imidazo[1,2-a]pyridine; m.p. 120—122°C.
24. 3-Amino-2-methyl-8-[E-1-(3-phenylpropen-1-yl)]-imidazo[1,2-a]pyridine; m.p. of hydrochloride: 222—224°C. (dec.)
25. 3-Ethylamino-2-methyl-8-(2-phenylethyl)-imidazo[1,2-a]pyridine; m.p. 145—147°C. (dec.)
26. 3-Amino-2,7-dimethyl-8-(2-phenylethyl)-imidazo[1,2-a]pyridine; m.p.
27. 8-Benzyloxy-3-isocyanomethyl-2-methyl-imidazo[1,2-a]pyridine; m.p.

Other typical compounds of this invention are listed in the Tables I to V below.

TABLE I

Compounds of the formula

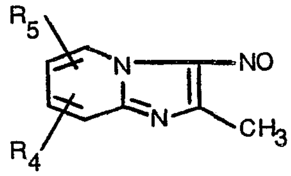

| Compound No. | $R_4$ | $R_5$ | m.p. °C. |
|---|---|---|---|
| 28. | $(8)-O-CH_2-$ 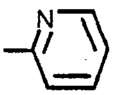 | H | |
| 29. | $(8)-CH=CH-$ 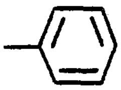 | H | (dark green solid) |
| 30. | $(8)-CH_2-CH_2-$ 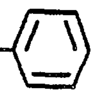 | H | 131.5 — 132.5 (dec.) |
| 31. | $(6)-CH_2-CH_2-$ 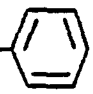 | H | 114 — 115 (dec.) |
| 32. | $(8)-O-CH_2-$ 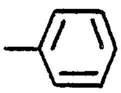 | H | 147.5 — 148.5 (dec.) |
| 33. | $(8)-O-CH_2-$ 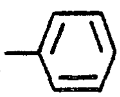 | (7)—$CH_3$ | 118 — 120 |
| 34. | $(8)-O-CH_2-$ 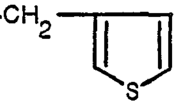 | H | 120 — 122 |
| 35. | $(7)-CH_2-CH_2-$ 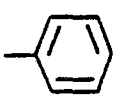 | H | 116 — 118 (dec.) |
| 36. | $(8)-CH=CH-$ 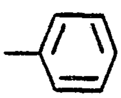 | H | 158 — 160 |
| 37. | $(8)-O-CH_2-$ 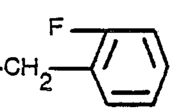 | H | 133 — 134 (dec.) |

TABLE II

Compounds of formula

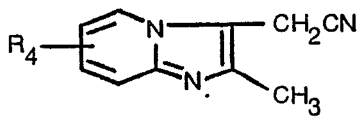

| Compound No. | $R_4$ | m.p. °C. |
|---|---|---|
| 38. | (8)–CH = CH 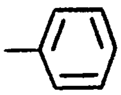 | 134 — 136 |
| 39. | (5)–$CH_2$–$CH_2$ 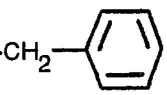 | 118 — 119 |
| 40. | (7)–$CH_2$–$CH_2$ 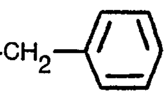 | 118 |
| 41. | (8)–O–$CH_2$–CH = $CH_2$ | 177 — 179 |

TABLE III

Compounds of formula

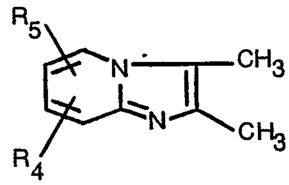

| Compound No. | $R_4$ | $R_5$ | m.p. °C. |
|---|---|---|---|
| 42. | (8)–CH = CH– 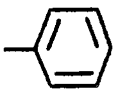 | (6)—$CH_3$ | 149 — 150 |
| 43. | cis (8)–CH = CH– 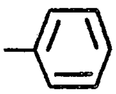 | H | 174 — 177 |
| 43A. | trans (8)–CH = CH– 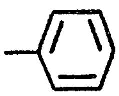 | H | 240 — 225 |
| 44. | (7)–$CH_2$–$CH_2$– 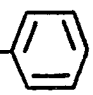 | H | 88 — 93 |
| 45. | (6)–$CH_2$–$CH_2$– 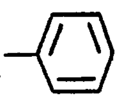 | H | 105 — 107 |
| 46. | (5)–$CH_2$–$CH_2$– 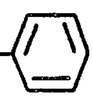 | H | 101 — 103 |
| 47. | (5)–O–$CH_2$– 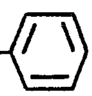 | H | 110 |
| 48. | (6)–$CH_2$– 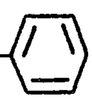 | H | 216 — 218 |

TABLE IV

Compounds of formula

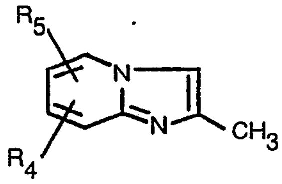

| Compound No. | $R_4$ | $R_5$ | m.p. °C. |
|---|---|---|---|
| 49. | (8)—CH = CH—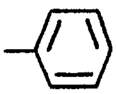 | (6)—$CH_3$ | 93 — 94 |
| 50. | (8)—CH = CH—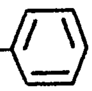 (cis) | H | (light yellow solid) |
| 51. | (8)—CH = CH—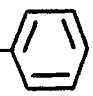 (trans) | H | 100 — 102 |
| 52. | (6)—$CH_2$—$CH_2$—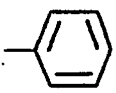 | H | 104 — 105 |
| 53. | (5)—$CH_2$—$CH_2$—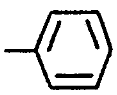 | H | 86 — 88 |
| 54. | (8)—CH = CH—$CH_2$—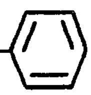 | H | 73.5 — 78 |
| 55. | (5)—O—$CH_2$—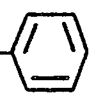 | H | (HCl-salt)<br>136.5 — 138.5 |
| 56. | (6)—O—$CH_2$—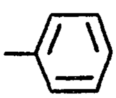 | H | (HBr-salt)<br>228 — 230 |
| 57. | (7)—$CH_2$—$CH_2$—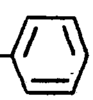 | H | (.HCl.1/4 $H_2O$)<br>92 — 122 |

TABLE V

Compounds of formula

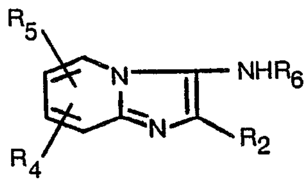

| Compound No. | $R_2$ | $R_4$ | $R_5$ | $R_6$ | m.p. °C. |
|---|---|---|---|---|---|
| 58 | $CH_3$ | (8)—O—$CH_2$— 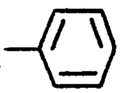 | H | H | (·$H_2PO_4$·1.33 $H_2O$) 214—215.5 dec. |
| 59. | $CH_3$ | (8)—O—$CH_2$— 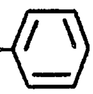 | H | $CH_3$ | (·HCl·0.25 $H_2O$) 193.5 — 194 |
| 60. | $C(CH_3)_3$ | (8)—O—$CH_2$— 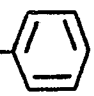 | H | H | 173 — 175 |
| 61. | $CH_3$ | (8)—O—$CH_2$—$CH_2$— 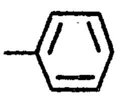 | H | H | (·HCl·1/4 $H_2O$) 138 — 140 dec. |
| 62. | $CH_3$ | (5)—$CH_2$—$CH_2$— 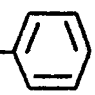 | H | H | (·HCl) 236 — 238 |
| 63. | $CH_3$ | (8)—$CH_2$—$CH_2$— 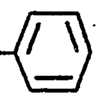 | (6)—$CH_3$ | H | (·HCl) 218 — 220 |
| 64. | $CH_3$ | (8)—O—$CH_2$— 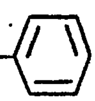 | (6)—Cl | H | 157 — 158 |
| 65. | $CH_2CH_2CH_2CH_3$ | (8)—O—$CH_2$— 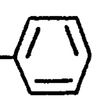 | H | H | 115 — 117 |
| 66. | $CH(CH_3)_2$ | (8)—O—$CH_2$— 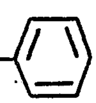 | H | H | 167 — 169 |

TABLE V (Continued)

| Compound No. | $R_2$ | $R_4$ | $R_5$ | $R_6$ | m.p. °C. |
|---|---|---|---|---|---|
| 67. | $CH_2CH_2CH_3$ | 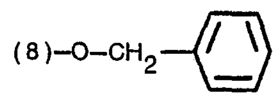 | H | H | 88 — 89 |
| 68. | $CH_3$ | 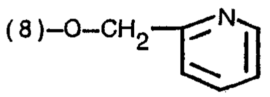 | H | H | 120 — 121 (dec.) |
| 69. | H | 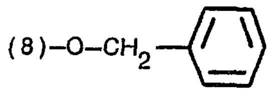 | H | H | (HCl) 209 — 210 |

Preferred imidazo[1,2-a]pyrazine compounds of this invention are:
8-benzyloxy-3-cyanomethyl-2-methyl-imidazo[1,2-a]pyrazine;
8-benzyloxy-2,3-dimethyl-imidazo[1,2-a]pyrazine;
3-amino-8-benzyloxy-2-methyl-imidazo[1,2-a]pyrazine; and
8-benzyloxy-3-ethylamino-2-methyl-imidazo[1,2-a]pyrazine.

The term "pharmaceutically acceptable salts" of this invention include salts wherein the acidic hydrogen forms an acid addition salt with an amine. (e.g. the phosphate salt of 3-amino-8-benzyloxy-2-methyl-imidazo[1,2-a]pyridine).

Suitable acids for the pharmaceutically acceptable acid addition salts include hydrochloric, sulfuric, phosphoric, nitric, acetic, propionic, maleic, ascorbic, citric and the like.

The acid addition salts are prepared via procedures well known in the art.

The compounds of this invention are useful in the treatment of peptic ulcers, having characteristics which enable the compounds to relieve the symptoms of peptic ulcer disease (including stress ulceration) and promote healing of gastic and/or duodenal ulcers. The anti-ulcer-activity of the compounds of Formula I is identified by tests which measure their gastic antisecretory activity in the rat and dog and by tests which measure their cytoprotective effect (sometimes also referred to in the art as mucoprotective effect) in rats. The compounds are useful as conjunctive therapeutic agents for co-administration with such anti-inflammatory/analgesic agents as aspirin, indomethacin, phenylbutazones, ibuprofen, naproxen, tolmetin and other agents having the untoward side effect of contributing to damage to the gastrointestinal tract.

The compounds of this invention, in order to be evaluated for their applied use characteristics undergo testing procedures according to standard biological procedures wherein the compounds are evaluated both on an absolute basis and on a comparative basis with compounds known to possess the characteristics useful for the treatment and/or prevention of peptic ulcer disease, duodenal ulcer disease and drug induced gastric ulceration. Such tests include testing in dogs prepared under aseptic surgical conditions with either Heidenhain gastric pouches or simple gastric fitulas fitted to facilitate collection of gastric secretions. The test compounds are administered in appropriate and well-defined and well-known vehicles for either intravenous delivery or oral administration. The agonists employed to stimulate gastric secretion include such compounds as histamine, pentagastrin, and feeding in dogs equipped with Heidenhain pouches, and insulin hypoglycemia (in addition to histamine and pentagastrin) in dogs with gastric fistulas.

Caesarean-derived Sprague-Dawley male rats are used for gastric secretion with pyloric ligation techniques and anti-ulcer studies employing aspirin-induced ulceration.

From these and other tests, as well as by comparison with known anti-ulcer agents the compounds of this invention are found to be effective for the oral treatment of the ulcerative disease states herein mentioned at doses of about 0.5 to 50 mgm per kilogram of body weight per day, preferably being administered in 3—4 divided doses per day. In those instances wherein it is desired to administer the compounds of this invention via a parenteral route (e.g. intravenously) the compounds are administered at a dose range of about 0.01 to 10 mg/kg in single or multiple daily doses. Of course, the dose will be regulated by the attending diagnostician depending on factors such as the degree and severity of the disease state and age and general condition of the patient being treated. The recommended dose range for

the preferred compounds of this invention is the oral dose of 75 to 1600 mg/day in three to four divided doses to achieve relief of the symptoms of peptic ulcer disease and promote the healing of gastric and/or duodenal ulcers.

In their use in the treatment of peptic ulcer disease, gastic and duodenal ulcers, and in the prevention and treatment of drug-induced gastic ulceration, the compounds are administered in unit dosage forms such as tablets, capsules, pills, powders, granules, sterile parenteral solutions or suspensions, suppositories and the like. Such preparations are prepared according to standard techniques well-known in the art. A few examples of such pharmaceutical formulations are as follows.

Formulations

The following formulations are to exemplify some of the dosage forms in which the anti-ulcer agents of this invention may be employed. In each, the active ingredient is designated by the term "Drug" which is meant to indicate one of the following compounds:

3-amino-2-methyl-8-(2-phenylethyl)-imidazo[1,2-a]pyridine;
2,3-dimethyl-8-(2-phenylethenyl)-imidazo[1,2-a]pyridine;
3-cyanomethyl-2-methyl-8-[E-1-(3-phenyl-propen-1-yl)]-imidazo[1,2-a]pyridine;
8-benzyloxy-3-amino-2-methyl-imidazo[1,2-a]pyrazine; and
8-benzyloxy-2,3-dimethyl-imidazo[1,2-a]pyrazine.

It will be appreciated, however, that each of these compounds may be replaced by equally effective quantities of other compounds defined by Formula I and their pharmaceutically acceptable salts.

Formulation 1

Tablets

| No. | Ingredient | mg/tab | mg/tab |
|---|---|---|---|
| 1 | Drug | 25.0 | 400 |
| 2 | Lactose impalpable powder USP | 114 | 241.5 |
| 3 | Corn starch USP | 25.0 | 50.0 |
| 4 | Corn starch as 5% paste in distilled water | 10.0 | 35.0 |
| 5 | Corn starch USP | 25.0 | 50.0 |
| 6 | Magnesium stearate USP | 1.00 | 3.50 |
| | | 200 | 780 |

Method of Manufacture

Mix items nos. 1, 2 and 3 in a suitable blender 5 to 15 minutes. Pass through a fine screen (#40) if necessary. Re-blend for 5 to 10 minutes and granulate with item no. 4. Pass the damp granulated mass through a coarse sieve (#6) using a suitable mill. Dry the damp granules at 40 to 50°C. overnight. Mill the dried granules using no. 20 screen. Add item no. 5 and blend for 5 to 10 minutes. Add item no. 6 and blend further for 3 to 5 minutes. Compress the tablet mixture into appropriate size and weight tablets with a suitable tabletting machine.

Formulation 2

Capsules

| No. | Ingredient | mg/tab | mg/tab |
|---|---|---|---|
| 1 | Drug | 25.0 | 400 |
| 2 | Lactose, impalpable powder USP | 144 | 191.5 |
| 3 | Corn starch USP | 30.0 | 105 |
| 4 | Magnesium stearate USP | 1.00 | 3.50 |
| | | 200 | 700 |

Method of Manufacture

Mix items nos. 1, 2 and 3 in a suitable blender for 5 to 10 minutes. Pass through a fine screen (#40) if necessary. Re-blend for 5 to 10 minutes, add item no. 4 and mix further for 3 to 50 minutes. Using a suitable machine, encapsulate the mixture into a two-piece hard gelatin capsule of appropriate size.

Formulation 3

Suspensions

| Ingredients | Formula A (mg/ml) | Formula B (mg/ml) |
|---|---|---|
| Drug | 5.0 | 80.0 |
| Sucrose | 600.0 | 600.0 |
| Benzyl alcohol | 10.0 | 10.0 |
| Methylcellulose (15 cps) | 4.0 | 4.0 |
| Polysorbte 80 | 5.0 | 5.0 |
| Vanillin | 0.2 | 0.2 |
| Purified Water q.s. | 1.0 ml | 1.0 ml |

Method of Manufacture

1. Charge approximately 40% of the final volume of purified water in a stainless steel tank. Heat to boiling. Agitate using an appropriate stirrer. Agitation should continue throughout procedure.
2. Add sucrose until it is dissolved.
3. Slowly add methylcellulose until it is well dispersed.
4. Start cooling the mixture to room temperature.
5. Add polysorbate, benzyl alcohol and vanillin until all ingredients are well dispersed.
6. Add the Drug until an uniform dispersion is formed.
7. This suspension is then q.s. to final volume with purified water at 25°C.

Formulation 4

Parenterals

| | mg/ml |
|---|---|
| Drug | 25.00 |
| Methylparaben | 1.30 |
| Propylparaben | 0.20 |
| Sodium bisulfite | 3.20 |
| Disodium edetate | 0.20 |
| Sodium sulfate | 2.60 |
| Water for injection q.s. ad | 1.0 ml |

Method for Manufacture

1. Dissolve parabens in a portion (approximately 85% of the final volume) of the water for injection at 65—70°C.
2. Cool to 25—35°C. Charge and dissolve sodium bisulfite, disodium edetate and sodium sulfate.
3. Charge and dissolve the Drug.
4. Bring the solution to the final volume by adding water for injection.
5. Filter the solution through 0.22 μ membrane and fill into appropriate containers.
6. Terminally sterilize the units by autoclaving.

## Formulation 5

Injectable Suspension

| | mg/ml |
|---|---|
| Drug (Sterile) | 50.0 |
| Benzyl alcohol | 9.0 |
| Methylparaben | 1.8 |
| Propylparaben | 0.2 |
| Sodium carboxymethylcellulose | 5.0 |
| Polyethylene Glycol 4000 | 10.0 |
| Povidone | 5.0 |
| Sodium Citrate | 15.0 |
| Disodium edetate | 0.1 |
| Water for injection | q.s. |
| To make | 1.0 ml |

Parenterals

Method of Preparation

1. Dissolve parabens in a portion of water for injection at 65—70°C.
2. Cool to 25—35°C. Charge and dissolve benzyl alcohol, sodium citrate, disodium edetate, PEG 4000, povidone and sodium carboxymethylcellulose.
3. Filter the solution and sterilize by autoclaving.
4. Make a slurry of the sterile active and pass it through a colloid mill.
5. Mix it well with solution from Step 3 and pass it through the mill.
6. Bring the suspension to the final volume/weight and fill into sterile containers.

## Formulation 6

Suppositories

| A. | Formula | mg/supp |
|---|---|---|
| | Drug | 5.0 |
| | Cocoa butter | 1995.0 |
| | | 2.0 g. |

Procedure

1. Melt cocoa butter to about 32—35°C.
2. Blend Drug into cocoa butter until well dispersed.
3. Pour into teflon-coated mould and congeal in refrigerator. Keep in refrigerator for an appropriate length of time.
4. Remove suppositories from mould.

| B. | Formula | mg/supp |
|---|---|---|
| | Drug | 100.0 |
| | PEG 1000 | 1824.0 |
| | PEG 4000 | 76 |
| | | 2.0 g. |

Procedure

1. Melt PEG 1000 and PEG 4000 in one container to 50°C.
2. Add Drug to the mixture. Blend until well dispersed.
3. Pour into mould and congeal in refrigerator. Keep in refrigerator for an appropriate length of time.
4. Remove suppositories from mould.

**Claims for the Contracting States: BE CH DE FR GB IT LI LU NL SE**

1. Imidazo[1,2-a]pyridines and pyrazines of the general formula

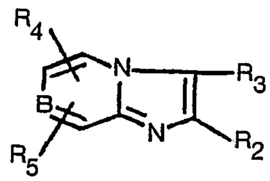

I

wherein B is CH or N, whereby, when B is CH, then
$R_2$ is hydrogen, $C_1$—$C_6$ alkyl or hydroxy $C_1$—$C_6$ alkyl;
$R_5$ is hydrogen, halogen or $C_1$—$C_6$ alkyl; and either
$R_3$ is $C_1$—$C_6$ alkyl, —$CH_2CN$, hydroxy $C_1$—$C_6$ alkyl, —NO,

$R_6$
/
—$CH_2NC$, —N
\
$R_7$

or, provided $R_2$ is not hydrogen, also hydrogen and
$R_4$, being attached to any of the positions 5-, 6- or 7- of the nucleus, represents one of the groupings

—O—$R_8$—Ar, —NH—$R_8$—Ar, —$R_8$—Ar, —CH=CH—Ar, —CH=CH—$CH_2$—Ar or —O—$CH_2$—CH=$CH_2$; or

$R_3$ is as defined above and
$R_4$, being attached to the 8-position of the nucleus, represents any one of the groupings:

—CH=CH—Ar, —CH=CH—$CH_2$—Ar or —O—$CH_2$—CH=$CH_2$; or

$R_6$
/
$R_3$ is —NO, —$CH_2NC$ or —N and
\
$R_7$

$R_4$, being attached to the 8-position of the nucleus, represents —O—$R_8$—Ar, —NH—$R_8$—Ar or —$R_8$—Ar; and when B is n, then
$R_5$ is as above defined,
$R_2$ and $R_3$ an independently selected from hydrogen, $C_1$—$C_6$ alkyl, hydroxy $C_1$—$C_6$ alkyl, —$CH_2CN$, —NO and —$NR_6R_7$ and
$R_4$ is —O—$R_8$—Ar, —NH—$R_8$—Ar, $R_8$—Ar, —CH=CH—Ar or —CH=CH—$CH_2$—Ar;
whereby, in the above definitions, $R_6$ and $R_7$ are independently selected from hydrogen or $C_1$—$C_6$ alkyl;
$R_8$ is a straight- or branched- chain $C_1$—$C_6$ alkylene group and
Ar represents thienyl, furanyl, pyridyl, phenyl or phenyl substituted by one or more substituents selected from halogen and $C_1$—$C_6$ alkyl;
the 2,3-dihydro; 5,6,7,8-tetrahydro and 2,3,5,6,7,8-hexahydro derivatives thereof and the pharmaceutically acceptable salts of such compounds.

2. Compounds of Formula I wherein B is CH,
$R_2$ represents —$CH_3$ or —$CH_2CH_3$;
$R_5$ represents hydrogen or —$CH_3$;
$CH_3$ represents —$NH_2$, —$NHC_2H_5$, —$CH_2CN$ or —$CH_3$ and
$R_4$, being attached to the 5-, 6- or 7- position of the nucleus, represents —O—$R_8$—Ar, —NH-$R_8$—Ar, —$R_8$—Ar, —CH=CH—Ar or —CH=CH—$CH_2$—Ar with $R_8$ being methylene, ethylene or propylene and Ar being phenyl, o-fluorophenyl, p-fluorophenyl, p-chlorophenyl, thienyl or furanyl.

3. Compounds of Formula I wherein B, $R_2$ and $R_5$ are as defined in claim 2; $R_4$, being attached to the 8-position of the nucleus, is as defined in claim 2 and $R_3$ represents —$NH_2$ or —$NHC_2H_5$.

4. Compounds of Formula I wherein B, $R_2$ and $R_5$ are as defined in claim 2, $R_3$ is —$CH_2CN$ or —$CH_3$ and $R_4$, being attached to the 8-position of the nucleus, is —CH=CH—Ar or —CH=CH—$CH_2$—Ar, with Ar being as defined in claim 2.

5. Compounds of the formula

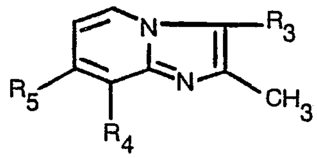

IA

wherein

$R_3$ is $-NH_2$;

$R_4$ is $-CH_2-CH_2-Ar$ or $-CH_2-CH_2-CH_2-Ar$ and

$R_5$ is hydrogen or methyl, whereby

Ar is phenyl or 3-thienyl.

6. Compounds of Formula IA set forth in claim 5, wherein

$R_3$ is $-NH_2$, $-CH_2CN$ or $-CH_3$

$R_4$ is $-CH{=}CH-Ar$ or $-CH{=}CH-CH_2-Ar$ and

$R_5$ is hydrogen or methyl, whereby

Ar is phenyl or 3-thienyl.

7. Compounds of the formula

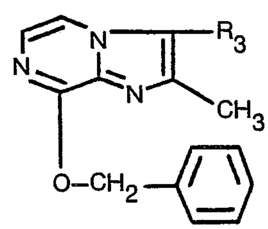

wherein $R_3$ is $C_1-C_6$ alkyl, $-CH_2CN$, $-NH_2$ or $-NHC_2H_5$.

8. Compound according to claim 5, being 3-amino-2-methyl-8-(2-phenylethyl)-imidazo[1,2-a]pyridine.

9. Compounds according to claim 6, being 2,3-dimethyl-8-(2-phenyl-ethenyl)-imidazo[1,2-a]pyridine and 3-cyanomethyl-2-methyl-8-[E-1-(3-phenyl-propen-1-yl)]-imidazo[1,2-a]pyridine.

10. Compounds according to claim 7, being 8-benzyloxy-3-cyanomethyl-2-methyl-imidazo[1,2-a]pyrazine; 8-benzyloxy-2,3-dimethyl-imidazo[1,2-a]pyrazine and 3-amino-8-benzyloxy-2-methyl-imidazo[1,2-a]pyrazine.

11. Pharmaceutical compositions comprising a compound as defined in any one of claims 1 to 10.

12. Process for the preparation of pharmaceutical composition as defined in claim 11, characterized in that a compound as defined in any one of claims 1 to 10 is admixed with one or more suitable pharmaceutical carriers.

13. Process for the preparation of compounds as defined in any one of claims 1 to 10, characterized in that either

A: a compound of the general formula

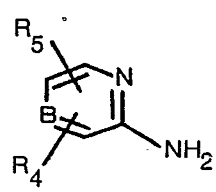

II

is reacted with a compound of the general formula

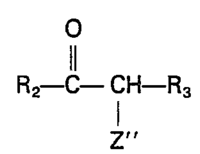

III

whereby, in the formulae, $R_2$, $R_3$, $R_4$ and $R_5$ are as defined in claim 1, except that any free amino or hydroxy groups present in $R_2$ or $R_3$ may be protected by a protecting group which is subsequently removed, and Z'' represents a good leaving group; or,

B: for preparation of compounds wherein $R_4$ is $—O—R_8—Ar$, $—O—CH_2—CH{=}CH_2$, $—NH—R_8—Ar$, $—R_8—Ar$, with $R_8$ and Ar being as defined in claim 1, a compound of the general formula

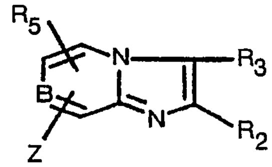

IV

is reacted with a compound of the general formula

Hal—Z'

V

whereby in the above formulae $R_2$, $R_3$ and $R_5$ are as defined in claim 1, Hal represents Br, Cl or J,

Z represents halogen, OH, or $NH_2$ and

Z' represents $—R_8—Ar$ or $—CH_2—CH{=}CH_2$.

C: for the preparation of a compounds wherein $R_4$ represents $—CH{=}CH—Ar$ or $—CH{=}CH—CH_2—Ar$, a compound of Formula IV wherein $R_2$, $R_3$ and $R_5$ are as defined in claim 1 and Z represents CHO is subjected to a Wittig reaction with the appropriate Wittig reagent; or

D: for the preparation of compounds of Formula I wherein $R_4$ represents $—CH{=}CH—Ar$, reacting a compound of Formula IV wherein $R_2$, $R_3$, and $R_5$ are as defined in claim 1 and Z represents a phosphinylmethyl group, with a compound of formula Ar—CHO whereby Ar is as defined above; or

E: for the preparation of a compound of Formula I wherein $R_2$, $R_4$ and $R_5$ are as defined in claim 1 and $R_3$ represents $CH_2CN$, reacting a compound of Formula I wherein $R_3$ is a group $—CH_2X$, X being a good leaving group, with a metal cyanide; or

F: for the preparation of a compound of Formula I wherein $R_2$, $R_3$, $R_4$ and $R_5$ are as defined in claim 1, except that at least one of $R_2$ and $R_3$ must be hydroxy $C_1—C_6$ alkyl, reducing a compound of Formula I wherein $R_2$ and/or $R_3$ is a group $—(R')_nCOOR$, R' being a $C_1—C_6$ alkylene group having 1 to 5 carbon atoms, n being zero or one and R being a hydrocarbon group; or

G: for the preparation of a compound of Formula I wherein $R_2$, $R_4$ and $R_5$ are as defined in claim 1 and $R_3$ is —NO, subjecting a compound of Formula I wherein $R_3$ is hydrogen to a nitrosation in the 3-position, or

H: for the preparation of compounds of Formula I wherein $R_3$ is $—NH_2$ and $R_2$, $R_4$ and $R_5$ are as defined in claim 1, reducing a compound of Formula I wherein $R_3$ is —NO or $NO_2$; or

I: for the preparation of a compound of Formula I wherein $R_2$, $R_4$ and $R_5$ are as defined in claim 1 and $R_3$ is $—NR_6R_7$ with $R_6$ and/or $R_7$ being $C_1—C_6$ alkyl, subjecting a compound of Formula I wherein $R_3$ is $—NH_2$ to an alkylation; or

J: for the preparation of a compound of Formula I wherein $R_2$, $R_4$ and $R_5$ are as defined in claim 1 and $R_3$ is $—CH_2NC$, subjecting a compound wherein $R_3$ is a group

$$—CH_2NH\overset{\displaystyle O}{\overset{\|}{C}}H$$

to a reaction with $PCl_3$ in the presence of an amine;

that a so-obtained compound of Formula I, if desired, is reduced to the corresponding 2,3-dihydro, 5,6,7,8-tetrahydro- or 2,3,5,6,7,8-hexahydro derivative;

and that a compound obtained according to any one of the processes described above, if desired, is tranformed into a salt.

14. Process according to claim 13, characterized in that in process

A: Z'' represents halogen, tosyl or mesyl and the reaction is carried out by heating the reactants together in an inert solvent;

B: when Z represents halogen, a copper catalyst is used;

C: the Wittig reagent used is of the formula

$$Ar—CH_2—\overset{O^{\ominus}}{\overset{|}{\underset{\oplus}{P}}}(OR)_2$$

with Ar being as defined in claim 1, R being a hydrocarbon group;

E: X represents halogen, alkoxy, aryloxy, mesyl, tosyl, a quaternary group, preferably $N^{\oplus}(CH_3)_3J^{\ominus}$, or a quaternary group wherein the quaternary ion is a non-nucleophilic counter ion selected from $BF_4^{\ominus}$, $PF_6^{\ominus}$, $CF_3SO_3^{\ominus}$, and $FSO_3^{\ominus}$ and the metal cyanide is an alkali metal cyanide;

F: the reduction is carried out by means of lithium aluminium hydride;

G: the nitrosation is carried out by means of a nitrite, preferably sodium nitrite, in the presence of concentrated HCl;

H: the reduction is carried out by means of zinc powder in acetic acid.

**Claims for the Contracting State: AT**

1. Process for the preparation of imidazo[1,2-a]pyridines and pyrazines of the general formula

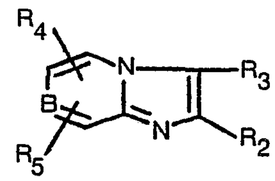

I

wherein B is CH or N, whereby, when B is CH, then

$R_2$ is hydrogen, $C_1$—$C_6$ alkyl or hydroxy $C_1$—$C_6$ alkyl;

$R_5$ is hydrogen, halogen or $C_1$—$C_6$ alkyl; and either

$R_3$ is $C_1$—$C_6$ alkyl, —$CH_2CN$, hydroxy $C_1$—$C_6$ alkyl, —NO,

$$—CH_2NC, —N\begin{matrix} R_6 \\ R_7 \end{matrix}$$

or, provided $R_2$ is not hydrogen, also hydrogen and

$R_4$, being attached to any of the positions 5-, 6- or 7- of the nucleus, represents one of the groupings

—O—$R_8$—Ar, —NH—$R_8$—Ar, —$R_8$—Ar, —CH=CH—Ar, —CH=CH—$CH_2$—Ar or —O—$CH_2$—CH=$CH_2$; or

$R_3$ is as defined above and

$R_4$, being attached to the 8-position of the nucleus, represents any one of the groupings:

—CH=CH—Ar, —CH=CH—$CH_2$—Ar or —O—$CH_2$—CH=$CH_2$; or

$$R_3 \text{ is } —NO, —CH_2NC \text{ or } —N\begin{matrix} R_6 \\ R_7 \end{matrix} \quad \text{and}$$

$R_4$, being attached to the 8-position of the nucleus, represents —O—$R_8$—Ar, —NH—$R_8$—Ar or —$R_8$—Ar;

and when B is n, then

$R_5$ is as above defined,

$R_2$ and $R_3$ an independently selected from hydrogen, $C_1$—$C_6$ alkyl, hydroxy $C_1$—$C_6$ alkyl, —$CH_2CN$, —NO and —$NR_6R_7$ and

$R_4$ is —O—$R_8$—Ar, —NH—$R_8$—Ar, $R_8$—Ar, —CH=CH—Ar or —CH=CH—$CH_2$—Ar;

whereby, in the above definitions, $R_6$ and $R_7$ are independently selected from hydrogen or $C_1$—$C_6$ alkyl;

$R_8$ is a straight- or branched- chain $C_1$—$C_6$ alkylene group and

Ar represents thienyl, furanyl, pyridyl, phenyl or phenyl substituted by one or more substituents selected from halogen and $C_1$—$C_6$ alkyl;

the 2,3-dihydro; 5,6,7,8-tetrahydro and 2,3,5,6,7,8-hexahydro derivatives thereof and the pharmaceutically acceptable salts of such compounds; characterized in that.

A: a compound of the general formula

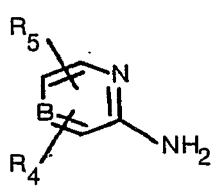

II

is reacted with a compound of the general formula

$$R_2—\overset{\overset{\large O}{\|}}{C}—\underset{\underset{\large Z''}{|}}{CH}—R_3$$

III

whereby, in the formulae, $R_2$, $R_3$, $R_4$ and $R_5$ are as defined in claim 1, except that any free amino or hydroxy groups present in $R_2$ or $R_3$ may be protected by a protecting group which is subsequently removed, and Z'' represents a good leaving group; or,

B: for preparation of compounds wherein $R_4$ is $—O—R_8—Ar$, $—O—CH_2—CH{=}CH_2$, $—NH—R_8—Ar$, $—R_8—Ar$, with $R_8$ and Ar being as defined in claim 1, a compound of the general formula

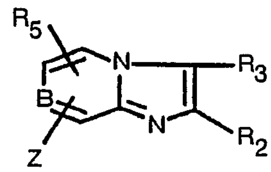

IV

is reacted with a compound of the general formula

$$Hal—Z' \qquad V$$

whereby in the above formulae $R_2$, $R_3$ and $R_5$ are as defined in claim 1, Hal represents Br, Cl or J,

Z represents halogen, OH, or $NH_2$ and

Z' represents $—R_8—Ar$ or $—CH_2—CH{=}CH_2$.

C: for the preparation of a compounds wherein $R_4$ represents $—CH{=}CH—Ar$ or $—CH{=}CH—CH_2—Ar$, a compound of Formula IV wherein $R_2$, $R_3$ and $R_5$ are as defined in claim 1 and Z represents CHO is subjected to a Wittig reaction with the appropriate Wittig reagent; or

D: for the preparation of compounds of Formula I wherein $R_4$ represents $—CH{=}CH—Ar$, reacting a compound of Formula IV wherein $R_2$, $R_3$, and $R_5$ are as defined in claim 1 and Z represents a phosphinylmethyl group, with a compound of formula Ar—CHO whereby Ar is as defined above; or

E: for the preparation of a compound of Formula I wherein $R_2$, $R_4$ and $R_5$ are as defined in claim 1 and $R_3$ represents $CH_2CN$, reacting a compound of Formula I wherein $R_3$ is a group $—CH_2X$, X being a good leaving group, with a metal cyanide; or

F: for the preparation of a compound of Formula I wherein $R_2$, $R_3$, $R_4$ and $R_5$ are as defined in claim 1, except that at least one of $R_2$ and $R_3$ must be hydroxyloweralkyl, reducing a compound of Formula I wherein $R_2$ and/or $R_3$ is a group $—(R')_nCOOR$, R' being a loweralkylene group having 1 to 5 carbon atoms, n being zero or one and R being a hydrocarbon group; or

G: for the preparation of a compound of Formula I wherein $R_2$, $R_4$ and $R_5$ are as defined in claim 1 and $R_3$ is —NO, subjecting a compound of Formula I wherein $R_3$ is hydrogen to a nitrosation in the 3-position, or

H: for the preparation of compounds of Formula I wherein $R_3$ is $—NH_2$ and $R_2$, $R_4$ and $R_5$ are as defined in claim 1, reducing a compound of Formula I wherein $R_3$ is —NO or $NO_2$; or

I: for the preparation of compounds of Formula I wherein $R_2$, $R_4$ and $R_5$ are as defined in claim 1 and $R_3$ is $—NR_6R_7$ with $R_6$ and/or $R_7$ being loweralkyl, subjecting a compound of Formula I wherein $R_3$ is $—NH_2$ to an alkylation; or

J: for the preparation of a compound of Formula I wherein $R_2$, $R_4$ and $R_5$ are as defined in claim 1 and $R_3$ is $—CH_2NC$, subjecting a compound wherein $R_3$ is a group

$$—CH_2NH\underset{\underset{O}{\|}}{C}H$$

to a reaction with $PCl_3$ in the presence of an amine;

that a so-obtained compound of Formula I, if desired, is reduced to the corresponding 2,3-dihydro, 5,6,7,8-tetrahydro- or 2,3,5,6,7,8-hexahydro derivative;

and that a compound obtained according to any one of the processes described above, if desired, is tranformed into a salt.

2. Process according to claim 1, characterized in that in process

A: Z'' represents halogen, tosyl or mesyl and the reaction is carried out by heating the reactants together in an inert solvent;

B: when Z represents halogen, a copper catalyst is used;

C: the Wittig reagent used is of the formula

$$Ar—CH_2—\overset{\overset{O^{\ominus}}{|}}{\underset{\oplus}{P}}(OR)_2$$

with Ar being as defined in claim 1, R being a hydrocarbon group;

E: X represents halogen, alkoxy, aryloxy, mesyl, tosyl, a quaternary group, preferably $N^{\oplus}(CH_3)_3J^{\ominus}$, or a

quaternary group wherein the quaternary ion is a non-nucleophilic counter ion selected from $BF_4^{\ominus}$, $PF_6^{\ominus}$, $CF_3SO_3^{\ominus}$, and $FSO_3^{\ominus}$ and the metal cyanide is an alkali metal cyanide;

F: the reduction is carried out by means of lithium aluminium hydride;

G: the nitrosation is carried out by means of a nitrite, preferably sodium nitrite, in the presence of concentrated HCl;

H: the reduction is carried out by means of zinc powder in acetic acid.

3. Process according to claim 1 or 2, characterized in that compounds of Formula I wherein B is CH,

$R_2$ represents $—CH_3$ or $—CH_2CH_3$;

$R_5$ represents hydrogen or $—CH_3$;

$R_3$ represents $—NH_2$, $—NHC_2H_5$, $—CH_2CN$ or $—CH_3$ and

$R_4$, being attached to the 5-, 6- or 7- position of the nucleus, represents $—O—R_8—Ar$, $—NH—R_8—Ar$, $—R_8—Ar$, $—CH{=}CH—Ar$ or $—CH{=}CH—CH_2—Ar$ with $R_4$ being methylene, ethylene or propylene and Ar being phenyl, o-fluorophenyl, p-fluorophenyl, p-chlorophenyl, thienyl or furanyl, are prepared.

4. Process according to claim 1 or 2, characterized in that compounds of Formula I wherein B, $R_2$ and $R_5$ are as defined in claim 3; $R_4$ being attached to the 8-position of the nucleus, is as defined in claim 3 and $R_3$ represents $—NH_2$ or $—NHC_2H_5$, are prepared..

5. Process according to claim 1 or 2, characterized in that compounds of Formula I wherein B, $R_2$ and $R_5$ are as defined in claim 3, $R_3$ is $—CHCN$ or $—CH_3$ and $R_4$, being attached to the 8-position of the nucleus, is $—CH{=}CH—Ar$ or $—CH{=}CH—CH_2—Ar$, with Ar being as defined in claim 3, are prepared.

6. Process according to claim 1 or 2, characterized in that compounds of the formula

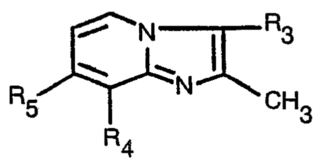

IA

wherein

$R_3$ is $—NH_2$;

$R_4$ is $—CH_2—CH_2—Ar$ or $—CH_2—CH_2—CH_2—Ar$ and

$R_5$ is hydrogen or methyl, whereby

Ar is phenyl or 3-thienyl, are prepared.

7. A process according to claim 1 or 2, characterized in that compounds of Formula IA set forth in claim 6, wherein

$R_3$ is $—NH_2$, $—CH_2CN$ or $—CH_3$

$R_4$ is $—CH{=}CH—Ar$ or $—CH{=}CH—CH_2—Ar$ and $R_5$ is hydrogen or methyl, whereby

Ar is phenyl or 3-thienyl, are prepared.

8. Process according to claim 1 or 2, characterized in that compounds of the formula

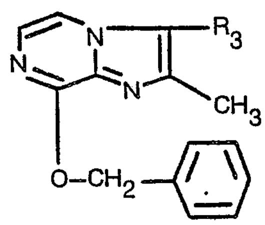

wherein $R_3$ is $C_1—C_6$ alkyl, $—CH_2CN$, $—NH_2$ or $—NHC_2Hs_5$, are prepared.

9. Process according to claim 1 or 2, characterized in that the compounds 3-amino-2-methyl-8-(2-phenylethyl)-imidazo[1,2-a]pyridine; 2,3-dimethyl-8-(2-phenyl-ethenyl)-imidazo[1,2-a]pyridine and 3-cyanomethyl-2-methyl-8-[E-1-(3-phenylpropen-1-yl)] imidazo[1,2-a]pyridine, are prepared.

10. Process according to claim 1 or 2, characterized in that the compounds 8-benzyloxy-3-cyanomethyl-2-methyl-imidazo[1,2-a]pyrazine; 8-benzyloxy-2,3-dimethyl-imdiazo[1,2-a]pyrazine and 3-amino-8-benzyloxy-2-methyl-imidazo[1,2-a]pyrazine are prepared.

**Patentansprüche für die Vertragsstaaten: BE CH DE FR GB IT LI LU NL SE**

1. Imidazo[1,2-a]pyridine und -pyrazine der allgemeinen Formel

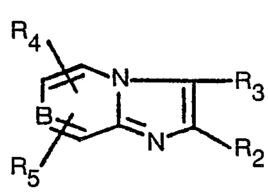

I

in der

B CH oder N ist, wobei, wenn B CH ist, dann

$R_2$ Wasserstoff, $C_1$—$C_6$-Alkyl oder Hydroxy-$C_1$—$C_6$-Alkyl ist;

$R_5$ Wasserstoff, Halogen oder $C_1$—$C_6$-Alkyl ist; und

entweder

$R_3$ $C_1$—$C_6$-Alkyl, —$CH_2CN$, Hydroxy-$C_1$—$C_6$-Alkyl, —NO, —$CH_2NC$,

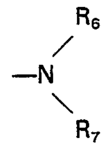

oder, vorausgesetzt, daß $R_2$ nicht Wasserstoff ist, auch Wasserstoff ist und

$R_4$, gebunden in einer der Stellungen 5-, 6- oder 7- des Kerns, eine der Gruppierungen —O—$R_8$—Ar, —NH—$R_8$—Ar, —$R_8$—Ar, —CH=CH—Ar, —CH=CH—$CH_2$—Ar oder —O—$CH_2$—CH=$CH_2$ bezeichnet;

oder

$R_3$ die im Vorstehenden angegebene Bedeutung hat und

$R_4$, gebunden in der Stellung 8- des Kerns, irgendeine der Gruppierungen —CH=CH—Ar, —CH=CH—$CH_2$—Ar oder —O—$CH_2$—CH=$CH_2$ bezeichnet;

oder

$R_3$ NO, —$CH_2NC$ oder

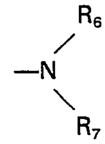

ist und

$R_4$ gebunden in der Stellung 8- des Kerns, —O—$R_8$—Ar, —NH—$R_8$—Ar oder —$R_8$—Ar bezeichnet, und wenn B N ist, dann

$R_5$ die oben angegebenen Bedeutungen hat,

$R_2$ und $R_3$ unabhängig aus Wasserstoff, $C_1$—$C_6$-Alkyl, Hydroxy-$C_1$—$C_6$-Alkyl, —$CH_2CN$, —NO und —$NR_6R_7$ ausgewählt sind und

$R_4$ —O—$R_8$—Ar, —NH—$R_8$—Ar, —$R_8$—Ar, —CH=CH—Ar oder —CH=CH—$CH_2$—Ar ist;

wobei in den vorstehenden Definitionen

$R_6$ und $R_7$ unabhängig aus Wasserstoff oder $C_1$—$C_6$-Alkyl ausgewählt sind;

$R_8$ eine geradkettige oder ketennverzweigte $C_1$—$C_6$-Alkylen-Gruppe ist und

Ar Thienyl, Furanyl, Pyridyl, Phenyl oder durch einen oder mehrere, aus Halogen und $C_1$—$C_6$-Alkyl ausgewählte Substituenten substituiertes Phenyl bezeichnet;

deren 2,3-Dihydro-, 5,6,7,8-Tetrahydro- und 2,3,5,6,7,8-Hexahydro-Derivate und die pharmazeutisch annehmbaren Salze derartiger Verbindungen.

2. Verbindungen der Formel I, dadurch gekennzeichnet, daß B CH ist;

$R_2$ —$CH_3$ oder —$CH_2CH_3$ bezeichnet;

$R_5$ Wasserstoff oder —$CH_3$ bezeichnet;

$R_3$ —$NH_2$, —$NHC_2H_5$, —$CH_2CN$ oder —$CH_3$ bezeichnet und

$R_4$, gebunden in der 5-, 6- oder 7-Stellung des Kerns, —O—$R_8$—Ar, —NH—$R_8$—Ar, —$R_8$—Ar, —CH=CH—Ar oder —CH=CH—$CH_2$—Ar bezeichnet, worin $R_8$ Methylen, Ethylen oder Propylen ist und Ar Phenyl, o-Fluorophenyl, p-Fluorophenyl, p-Chlorophenyl, Thienyl oder Furanyl ist.

3. Verbindungen der Formel I, dadurch gekennzeichnet, daß B, $R_2$ und $R_5$ die in Anspruch 2 angegebenen Bedeutungen haben, $R_4$, gebunden in der Stellung 8- des Kerns, die in Anspruch 2 angegebenen Bedeutungen hat und $R_3$ —$NH_2$ oder —$NHC_3H_5$ bezeichnet.

4. Verbindungen der Formel I, dadurch gekennzeichnet, daß B, $R_2$ und $R_5$ die in Anspruch 2 angegebenen Bedeugungen haben, $R_3$ —$CH_2CN$ oder —$CH_3$ ist und $R_4$, gebunden in der Stellung 8- des Kerns, —CH=CH—Ar oder —CH=CH—$CH_2$—Ar ist, worin Ar die in Anspruch 2 angegebenen Bedeutungen hat.

5. Verbindungen der Formel

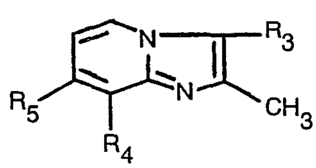

IA

in der

$R_3$ —$NH_2$ ist;

$R_4$ —$CH_2$—$CH_2$—Ar oder —$CH_2$—$CH_2$—$CH_2$—Ar ist und

$R_5$ Wasserstoff oder Methyl ist, worin
Ar Phenyl oder 3-Thienyl ist.

6. Verbindungen der Formel IA nach Anspruch 5, dadurch gekennzeichnet, daß
$R_3$ —$NH_2$, —$CH_2CN$ oder —$CH_3$ ist;
$R_4$ —CH=CH—Ar oder —CH=CH—$CH_2$—Ar ist und
$R_5$ Wasserstoff oder Methyl ist, worin
Ar Phenyl oder 3-Thienyl ist.

7. Verbindungen der Formel

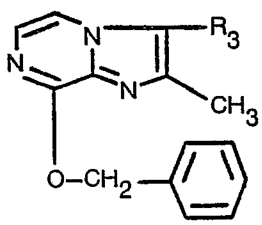

in der
$R_3$ $C_1$—$C_6$-Alkyl, —$CH_2CN$, —$NH_2$ oder —$NHC_2H_5$ ist.

8. Verbindung nach Anspruch 5, die 3-Amino-2-methyl-8-(2-phenylethyl)-imidazo[1,2-a]pyridin ist.

9. Verbindungen nach Anspruch 6, die 2,3 - Dimethyl - 8 - (2 - phenyl - ethenyl) - imidazo[1,2 - a]-pyridin und 3 - Cyanomethyl - 2 - methyl - 8 - [E - 1 - (3 - phenyl - propen - 1 - yl)]imidazo[1,2 - a]-pyridin sind.

10. Verbindungen nach Anspruch 7, die 8 - Benzyloxy - 3 - cyanomethyl - 2 - methyl-]imidazo - [1,2 - a]pyrazin, 8 - Benzyloxy - 2,3 - dimethyl-]imidazo[1,2 - a]pyrazin und 3 - Amino - 8 - benzyloxy - 2 - methyl - imidazo[1,2 - a]pyrazin sind.

11. Pharmazeutische Zusammensetzungen, enthaltend eine Verbindung nach irgendeinem der Ansprüche 1 bis 10.

12. Verfahren zur Herstellung pharmazeutischer Zusammensetzungen nach Anspruch 11, dadurch gekennzeichnet, daß eine Verbindung nach irgendeinem der Ansprüche 1 bis 10 mit einem oder mehreren geeigneten pharmazeutischen Trägern vermischt wird.

13. Verfahren zur Herstellung von Verbindungen nach irgendeinem der Ansprüche 1 bis 10, dadurch gekennzeichnet, daß entweder

A: eine Verbindung der allgemienen Formel

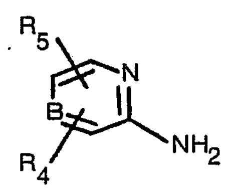

II

mit einer Verbindung der allgemeinen Formel

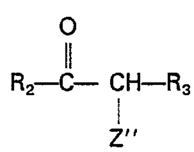

III

umgesetzt wird, wobei in den Formeln $R_2$, $R_3$, $R_4$ und $R_5$ die in Anspruch 1 angegebenen Bedeutungen haben, mit der Ausnahme, daß in $R_2$ oder $R_3$ vorhandene freie Amino- oder Hydroxy-Gruppen durch eine Schutzgruppe geschützt sein können, die anschließend entfernt wird, und Z'' eine leicht abspaltbare Gruppe bezeichnet, oder

B: zur Herstellung von Verbindungen, in denen $R_4$ —O—$R_8$—Ar, —O—$CH_2$—CH=$CH_2$, —NH—$R_8$—Ar, —$R_8$—Ar ist, worin $R_8$ und Ar die in Anspruch 1 angegebenen Bedeutungen haben, eine Verbindung der allgemeinen Formel

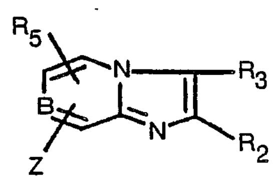

IV

mit einer Verbindung der allgemeinen Formel

Hal—Z'

umgesetzt wird, wobei in den vorstehenden Formeln $R_2$, $R_3$ und $R_5$ die in Anspruch 1 angegebenen Bedeutungen haben, Hal Br, Cl oder I bezeichnet, Z Halogen, OH oder $NH_2$ bezeichnet und Z' —$R_8$—Ar oder —$CH_2$—CH=$CH_2$ bezeichnet,

C: zur Herstellung von Verbindungen der Formel I, in denen $R_4$ —CH=CH—Ar oder —CH=CH—$CH_2$—Ar bezeichnet, eine Verbindung der Formel IV, in der $R_2$, $R_3$ und $R_5$ die in Anspruch 1 angegebenen Bedeutungen haben und Z CHO bezeichnet, einer Wittig-Reaktion mit dem passenden Wittig-Reagens unterworfen wird; oder

D: zur Herstellung von Verbindungen der Formel I, in denen $R_4$ —CH=CH—Ar bezeichnet, eine Verbindung der Formel IV, in der $R_2$, $R_3$ und $R_5$ die in Anspruch 1 angegebenen Bedeutungen haben und Z eine Phosphinylmethyl-Gruppe bezeichnet, mit einer Verbindung der Formel Ar—CHO, in der Ar die im Vorstehenden angegebene Bedeutung hat, umgesetzt wird; oder

E: zur Herstellung einer Verbindung der Formel I, in der $R_2$, $R_4$ und $R_5$ die in Anspruch 1 angegebenen Bedeutungen haben und $R_3$ $CH_2CN$ bezeichnet, eine Verbindung der Formel I, in der $R_3$ eine Gruppe —$CH_2X$ bezeichnet, worin X eine leicht abspaltbare Gruppe bezeichnet, mit einem Metallcyanid umgesetzt wird; oder

F: zur Herstellung von Verbindungen der Formel I, in der $R_2$, $R_3$, $R_4$ und $R_5$ die in Anspruch 1 angegebenen Bedeutungen haben, jedoch mit der Ausnahme, daß wenigstens einer der Substituenten $R_2$ und $R_3$ Hydroxy-$C_1$—$C_6$-alkyl sein muß, eine Verbindung der Formel I, in der $R_2$ und/oder $R_3$ eine Gruppe —$(R')_n$COOR ist, worin R' eine $C_1$—$C_6$-Alkylen-Gruppe mit 1 bis 5 Kohlenstoff-Atomen ist, n 0 oder 1 ist und R eine Kohlenwasserstoff-Gruppe ist, reduziert wird; oder

G: zur Herstellung von Verbindungen der Formel I, in der $R_2$, $R_4$ und $R_5$ die in Anspruch 1 angegebenen Bedeutungen haben und $R_3$ —NO bezeichnet, eine Verbindung der Formel I, in der $R_3$ Wasserstoff ist, einer Nitrosierung in der 3-Stellung unterworfen wird; oder

H: zur Herstellung von Verbindungen der Formel I, in der $R_3$ —$NH_2$ ist und $R_2$, $R_4$ und $R_5$ die in Anspruch 1 angegebenen Bedeutungen haben, eine Verbindung der Formel I, in der $R_3$ —NO oder —$NO_2$ bezeichnet, reduziert wird; oder

I: zur Herstellung von Verbindungen der Formel I, in der $R_2$, $R_4$ und $R_5$ die in Anspruch 1 angegebenen Bedeutungen haben und $R_3$ —$NR_6R_7$ ist, worin $R_6$ und $R_7$ $C_1$—$C_6$-Alkyl sind, eine Verbindung der Formel I, in der $R_3$ —$NH_2$ ist, einer Alkylierung unterworfen wird; oder

J: zur Herstellung einer Verbindung der Formel I, in der $R_2$, $R_4$ und $R_5$ die in Anspruch 1 angegebenen Bedeutungen haben und $R_3$ $CH_2NC$ bezeichnet, eine Verbindung der Formel I, in der $R_3$ eine Gruppe

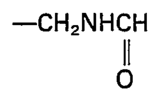

einer Reaktion mit $PCl_3$ in Gegenwart eines Amins unterworfen wird; daß eine auf diese Weise erhaltene Verbindung der Formel I gewünschtenfalls zu dem entsprechenden 2,3-Dihydro-, 5,6,7,8-Tetrahydro oder 2,3,5,6,7,8-Hexahydro-Derivat reduziert wird; und daß eine nach irgendeinem der im Vorstehenden beschriebenen Verfahren hergestellte Verbindung gewünschtenfalls in ein Salz überführt wird.

14. Verfahren nach Anspruch 13, dadurch gekennzeichnet, daß in dem Verfahren

A: Z'' Halogen, Tosyl oder Mesyl bezeichnet und die Reaktion durch gemeinsames Erhitzen der Reaktionspartner in einem inerten Lösungsmittel durchgeführt wird;

B: wenn Z Halogen bezeichnet, ein Kupfer-Katalysator verwendet wird;

C: das verwendete Wittig-Reagens die Formel

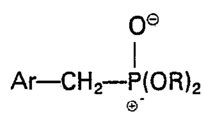

besitzt, in der Ar die in Anspruch 1 angegebene Bedeutung hat und R eine Kohlenwasserstoff-Gruppe ist;

E: X Halogen, Alkyloxy, Aryloxy, Mesyl, Tosyl, eine quaternäre Gruppe, vorzugsweise $\overset{\oplus}{N}(CH_3)_3\, I^{\ominus}$, oder eine quaternäre Gruppe, in der das quaternäre Ion ein nicht-nucleophiles Gegenion ausgewählt aus $BF_4^{\ominus}$, $PF_6^{\ominus}$, $CF_3SO_3^{\ominus}$ und $FSO_3^{\ominus}$ ist, bezeichnet, und das Metallcyanid ein Alkalimetallcyanid ist;

F: die Reduktion mit Hilfe von Lithiumaluminiumhydrid durchgeführt wird;

G: die Nitrosierung mit Hilfe eines Nitrits, vorzugsweise von Natriumnitrit, in Gegenwart konzentrierter HCl durchgeführt wird;

H: die Reduktion mit Hilfe von Zink-Pulver in Essigsäure durchgeführt wird.

**Patentansprüche für den Vertragsstaat: AT**

1. Verfahren zur Herstellung von Imidazo[1,2-a]pyridinen und -pyrazinen der allgemeinen Formel

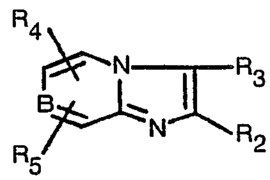

I

in der

B CH oder N ist, wobei, wenn B CH ist, dann

$R_2$ Wasserstoff, $C_1$—$C_6$-Alkyl oder Hydroxy-$C_1$—$C_6$-Alkyl ist;

$R_5$ Wasserstoff, Halogen oder $C_1$—$C_6$-Alkyl sit; und

entweder

$R_3$ $C_1$—$C_6$-Alkyl, —$CH_2CN$, Hydroxy-$C_1$—$C_6$-Alkyl, —NO, —$CH_2NC$,

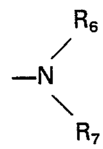

oder, vorausgesetzt, daß $R_2$ nicht Wasserstoff ist, auch Wasserstoff ist und

$R_4$, gebunden in einer der Stellungen 5-, 6- oder 7- des Kerns, eine der Gruppierungen —O—$R_8$—Ar, —NH—$R_8$—Ar, —$R_8$—Ar, —CH=CH—Ar, —CH=CH—$CH_2$—Ar oder —O—$CH_2$—CH=$CH_2$ bezeichnet;

oder

$R_3$ die im Vorstehenden angegebene Bedeutung hat und

$R_4$, gebunden in der Stellung 8- des Kerns, irgendeine der Gruppierungén —CH=CH—Ar, —CH=CH—$CH_2$—Ar oder —O—$CH_2$—CH=$CH_2$ bezeichnet;

oder

$R_3$ NO, —$CH_2NC$ oder

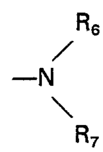

ist und

$R_4$ gebunden in der Stellung 8- des Kerns, —O—$R_8$—Ar, —NH—$R_8$—Ar oder —$R_8$—Ar bezeichnet, und wenn B N ist, dann

$R_5$ die oben angegebenen Bedeutungen hat,

$R_2$ und $R_3$ unabhängig aus Wasserstoff, $C_1$—$C_6$-Alkyl, Hydroxy-$C_1$—$C_6$-Alkyl, —$CH_2CN$, —NO und —$NR_6R_7$ ausgewählt sind und

$R_4$ —O—$R_8$—Ar, —NH—$R_8$—Ar, —$R_8$—Ar, —CH=CH—Ar oder —CH=CH—$CH_2$—Ar ist;

wobei in den vorstehenden Definitionen

$R_6$ und $R_7$ unabhängig aus Wasserstoff oder $C_1$—$C_6$-Alkyl ausgewählt sind;

$R_8$ eine geradkettige oder kentenverzweigte $C_1$—$C_6$-Alkylen-Gruppe ist und

Ar Thienyl, Furanyl, Pyridyl, Phenyl oder durch einen oder mehrere, aus Halogen und $C_1$—$C_6$-Alkyl ausgewählte Substituenten substituiertes Phenyl bezeichnet;

deren 2,3-Dihydro-, 5,6,7,8-Tetrahydro- und 2,3,5,6,7,8-Hexahydro-Derivaten und den pharmazeutisch annehmbaren Salze derartiger Verbindungen, dadurch gekennzeichnet, daß

A: eine Verbindung der allgemienen Formel

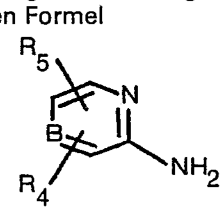

II

mit einer Verbindung der allgemeinen Formel

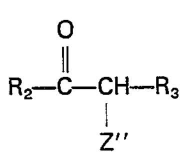

III

umgesetzt wird, wobei in den Formeln $R_2$, $R_3$, $R_4$ und $R_5$ die im Vorstehenden angegebenen Bedeutungen haben, mit der Ausnahme, daß in $R_2$ oder $R_3$ vorhandene freie Amino- oder Hydroxy-Gruppen durch eine Schutzgruppe geschützt sein können, die anschließend entfernt wird, und Z'' eine leicht abspaltbare Gruppe bezeichnet, oder

B: zur Herstellung von Verbindungen, in denen $R_4$ —O—$R_8$—Ar, —O—$CH_2$—CH=$CH_2$, —NH—$R_8$—Ar, —$R_8$—Ar ist, worin $R_8$ und Ar die im Vorstehenden angegebenen Bedeutungen haben, eine Verbindung der allgemeinen Formel

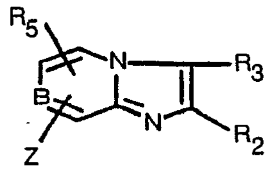

IV

mit einer Verbindung der allgemeinen Formel

Hal—Z'

umgesetzt wird, wobei in den vorstehenden Formeln $R_2$, $R_3$ und $R_5$ die im Vorstehenden angegebenen Bedeutungen haben, Hal Br, Cl oder I bezeichnet, Z Halogen, OH oder $NH_2$ bezeichnet und Z' —$R_8$—Ar oder —$CH_2$—CH=$CH_2$ bezeichnet,

C: zur Herstellung von Verbindungen der Formel I, in denen $R_4$ —CH=CH—Ar oder —CH=CH—$CH_2$—Ar bezeichnet, eine Verbindung der Formel IV, in der $R_2$, $R_3$ und $R_5$ die im Vorstehenden angegebenen Bedeutungen haben und Z CHO bezeichnet, einer Wittig-Reaktion mit dem passenden Wittig-Reagens unterworfen wird; oder

D: zur Herstellung von Verbindungen der Formel I, in denen $R_4$ —CH=CH—Ar bezeichnet, eine Verbindung der Formel IV, in der $R_2$, $R_3$ und $R_5$ die im Vorstehenden angegebenen Bedeutungen haben und Z eine Phosphinylmethyl-Gruppe bezeichnet, mit einer Verbindung der Formel Ar—CHO, in der Ar die im Vorstehenden angegebene Bedeutung hat, umgesetzt wird; oder

E: zur Herstellung einer Verbindung der Formel I, in der $R_2$, $R_4$ und $R_5$ die im Vorstehenden angegebenen Bedeutungen haben und $R_3$ $CH_2CN$ bezeichnet, eine Verbindung der Formel I, in der $R_3$ eine Gruppe —$CH_2X$ bezeichnet, worin X eine leicht abspaltbare Gruppe bezeichnet, mit einem Metallcyanid umgesetzt wird; oder

F: zur Herstellung von Verbindungen der Formel I, in der $R_2$, $R_3$, $R_4$ und $R_5$ die im Vorstehenden angegebenen Bedeutungen haben, jedoch mit der Ausnahme, daß wenigstens einer der Substituenten $R_2$ und $R_3$ Hydroxy-$C_1$—$C_6$-alkyl sein muß, eine Verbindung der Formel I, in der $R_2$ und/oder $R_3$ eine Gruppe —$(R')_n$COOR ist, worin R' eine $C_1$—$C_6$-Alkylen-Gruppe mit 1 bis 5 Kohlenstoff-Atomen ist, n 0 oder 1 ist und R eine Kohlenwasserstoff-Gruppe ist, reduziert wird; oder

G: zur Herstellung von Verbindungen der Formel I, in der $R_2$, $R_4$ und $R_5$ die im Vorstehenden angegebenen Bedeutungen haben und $R_3$ —NO bezeichnet, eine Verbindung der Formel I, in der $R_3$ Wasserstoff ist, einer Nitrosierung in der 3-Stellung unterworfen wird; oder

H: zur Herstellung von Verbindungen der Formel I, in der $R_3$ —$NH_2$ ist und $R_2$, $R_4$ und $R_5$ die im Vorstehenden angegebenen Bedeutungen haben, eine Verbindung der Formel I, in der $R_3$ —NO oder —$NO_2$ bezeichnet, reduziert wird; oder

I: zur Herstellung von Verbindungen der Formel I, in der $R_2$, $R_4$ und $R_5$ die im Vorstehenden angegebenen Bedeutungen haben und $R_3$ —$NR_6R_7$ ist, worin $R_6$ und $R_7$ $C_1$—$C_6$-Alkyl sind, eine Verbindung der Formel I, in der $R_3$ —$NH_2$ ist, einer Alkylierung unterworfen wird; oder

J: zur Herstellung einer Verbindung der Formel I, in der $R_2$, $R_4$ und $R_5$ die im Vorstehenden angegebenen Bedeutungen haben und $R_3$ $CH_2NC$ bezeichnet, eine Verbindung der Formel I, in der $R_3$ eine Gruppe

$$—CH_2NHCH(=O)$$

einer Reaktion mit $PCl_3$ in Gegenwart eines Amins unterworfen wird; daß eine auf diese Weise erhaltene Verbindung der Formel I gewünschtenfalls zu dem entsprechenden 2,3-Dihydro-, 5,6,7,8-Tetrahydro oder 2,3,5,6,7,8-Hexahydro-Derivat reduziert wird; und daß eine nach irgendeinem der im Vorstehenden beschriebenen Verfahren hergestellte Verbindung gewünschtenfalls in ein Salz überführt wird.

2. Verfahren nach Anspruch 1, dadurch gekennzeichnet, daß in dem Verfahren

A: Z'' Halogen, Tosyl oder Mesyl bezeichnet und die Reaktion durch gemeinsames Erhitzen der Reaktionspartner in einem inerten Lösungsmittel durchgeführt wird;

B: wenn Z Halogen bezeichnet, ein Kupfer-Katalysator verwendet wird;

C: das verwendete Wittig-Reagens die Formel

$$Ar-CH_2-\overset{O^{\ominus}}{\underset{\oplus}{P}}(OR)_2$$

besitzt, in der Ar die in Anspruch 1 angegebene Bedeutung hat und R eine Kohlenwasserstoff-Gruppe ist;

E: X Halogen, Alkyloxy, Aryloxy, Mesyl, Tosyl, eine quaternäre Gruppe, vorzugsweise $\overset{\oplus}{N}(CH_3)_3\ I^{\ominus}$, oder eine quaternäre Gruppe, in der das quaternäre Ion ein nicht-nucleophiles Gegenion ausgewählt aus $BF_4^{\ominus}$, $PF_6^{\ominus}$, $CF_3SO_3^{\ominus}$ und $FSO_3^{\ominus}$ ist, bezeichnet, und das Metallcyanid ein Alkalimetallcyanid ist;

F: die Reduktion mit Hilfe von Lithiumaluminiumhydrid durchgeführt wird;

G: die Nitrosierung mit Hilfe eines Nitrits, vorzugsweise von Natriumnitrit, in Gegenwart konzentrierter HCl durchgeführt wird;

H: die Reduktion mit Hilfe von Zink-Pulver in Essigsäure durchgeführt wird.

3. Verfahren nach Ansprüchen 1 oder 2, dadurch gekennzeichnet, daß Verbindungen der Formel I, in der B CH ist;

$R_2$ $—CH_3$ oder $—CH_2CH_3$ bezeichnet;

$R_5$ Wasserstoff oder $—CH_3$ bezeichnet;

$R_3$ $—NH_2$, $—NHC_2H_5$, $—CH_2CN$ oder $—CH_3$ bezeichnet und

$R_4$, gebunden in der 5-, 6- oder 7-Stellung des Kerns, $—O—R_8—Ar$, $—NH—R_8—Ar$, $—R_8—Ar$, $—CH{=}CH—Ar$ oder $—CH{=}CH—CH_2—Ar$ bezeichnet, worin $R_8$ Methylen, Ethylen oder Propylen ist und Ar Phenyl, o-Fluorophenyl, p-Fluorophenyl, p-Chlorophenyl, Thienyl oder Furanyl ist,

hergestellt werden.

4. Verfahren nach Ansprüchen 1 oder 2, dadurch gekennzeichnet, daß Verbindungen der Formel I, in der B, $R_2$ und $R_5$ die in Anspruch 3 angegebenen Bedeutungen haben, $R_4$, gebunden in der Stellung 8- des Kerns, die in Anspruch 3 angegebenen Bedeutungen hat und $R_3$ $—NH_2$ oder $—NHC_2H_5$ bezeichnet, hergestellt werden.

5. Verfahren nach Ansprüchen 1 oder 2 dadurch gekennzeichnet, daß Verbindungen der Formel I, in der B, $R_2$ und $R_5$ die in Anspruch 3 angegebenen Bedeutungen haben, $R_3$ $—CH_2CN$ oder $—CH_3$ ist und $R_4$, gebunden in der Stellung 8- des Kerns, $—CH{=}CH—Ar$ oder $—CH{=}CH—CH_2—Ar$ ist, worin Ar die in Anspruch 3 angegebenen Bedeutungen hat, hergestellt werden.

6. Verfahren nach Ansprüchen 1 oder 2, dadurch gekennzeichnet, daß Verbindungen der Formel

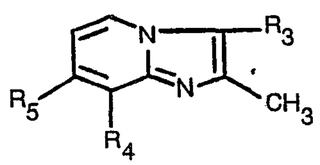

IA

in der

$R_3$ $—NH_2$ ist;

$R_4$ $—CH_2—CH_2—Ar$ oder $—CH_2—CH_2—CH_2—Ar$ ist und

$R_5$ Wasserstoff oder Methyl ist, worin

Ar Phenyl oder 3-Thienyl ist,

hergestellt werden.

7. Verfahren nach Ansprüchen 1 oder 2, dadurch gekennzeichnet, daß Verbindungen der Formel IA nach Anspruch 5, dadurch gekennzeichnet, daß

$R_3$ $—NH_2$, $—CH_2CN$ oder $—CH_3$ ist;

$R_4$ $—CH{=}CH—Ar$ oder $—CH{=}CH—CH_2—Ar$ ist und

$R_5$ Wasserstoff oder Methyl ist, worin

Ar Phenyl oder 3-Thienyl ist,

hergestellt werden.

8. Verfahren nach Ansprüchen 1 oder 2, dadurch gekennzeichnet, daß Verbindungen der Formel

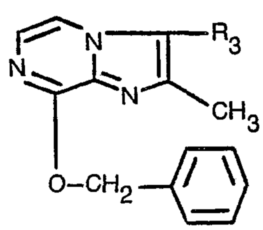

in der

$R_3$ $C_1—C_6$-Alkyl, $—CH_2CN$, $—NH_2$ oder $—NHC_2H_5$ ist, hergestellt werden.

9. Verfahren nach Ansprüchen 1 oder 2, dadurch gekennzeichnet, daß die Verbindungen, 3 - Amino - 2 - methyl - 8 - (2 - phenylethyl) - imidazo[1,2 - a]pyridin, 2,3 - Dimethyl - 8 - (2 - phenyl - ethenyl) - imidazo[1,2 - a]pyridin und 3 - Cyanomethyl - 2 - methyl - 8 - [E - 1 - (3 - phenyl - propen - 1 - yl)] - imidazo[1,2 .- a]pyridin hergestellt werden.

10. Verfahren nach Ansprüchen 1 oder 2, dadurch gekennzeichnet, daß die Verbindung, 8 - Benzyloxy - 3 - cyanomethyl - 2 - methyl-]imidazo[1,2 - a]pyrazin, 8 - Benzyloxy - 2,3 - dimethyl-] - imidazo[1,2 - a]pyrazin und 3 - Amino - 8 - benzyloxy - 2 - methyl - imidazo[1,2 - a]pyrazin hergestellt werden.

**Revendications pour les Etats contractants: BE CH DE FR GB IT LI LU NL SE**

1. Imidazo[1,2-a]pyridines et pyrazines de formule générale

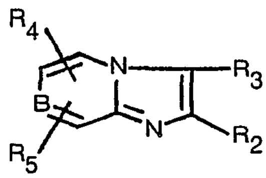

I

où B est CH ou N, et quand B est CH, alors

$R_2$ est hydrogène, alcoyle $C_1$—$C_6$ ou hydroxyalcoyle $C_1$—$C_6$;

$R_5$ est hydrogène, halogène ou alcoyle $C_1$—$C_6$;

et *soit*

$R_3$ est alcoyle $C_1$—$C_6$, —$CH_2CN$, hydroxyalcoyle $C_1$—$C_6$, —NO, —$CH_2NC$,

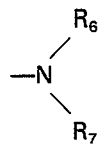

ou, à condition que $R_2$ ne soit pas de l'hydrogène, également de l'hydrogène et

$R_4$, étant attaché à toute position 5-, 6- ou 7- du noyau, représente l'un des groupements —O—$R_8$—Ar, —NH—$R_8$—Ar, —$R_8$—Ar, —CH=CH—Ar, —CH=CH—$CH_2$—Ar ou —O—$CH_2$—CH=$CH_2$; ou

$R_3$ est tel que défini ci-dessus et

$R_4$, étant attaché à la position 8 du noyau, représente l'un des groupements: —CH=CH—Ar, —CH=CH—$CH_2$—Ar ou —O—$CH_2$—CH=$CH_2$; ou

$R_3$ est —NO, —$CH_2NC$ ou

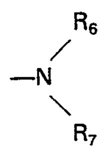

et

$R_4$, étant attaché à la position 8 du noyau, représente —O—$R_8$—Ar, —NH—$R_8$—Ar ou —$R_8$—Ar;

et quand B est N, alors

$R_5$ est tel que défini ci-dessus,

$R_2$ et $R_3$ sont indépendamment choisis parmi l'hydrogène, un alcoyle $C_1$—$C_6$, un hydroxyalcoyle $C_1$—$C_6$, —$CH_2CN$, —NO et —$NR_6R_7$ et

$R_4$ est —O—$R_8$—Ar, —NH—$R_8$—Ar, —$R_8$—Ar, —CH=CH—Ar ou —CH=CH—$CH_2$—Ar;

et dans les définitions ci-dessus, $R_6$ et $R_7$ sont indépendamment choisis parmi l'hydrogène ou un alcoyle $C_1$—$C_6$;

$R_8$ est un groupe alcoylène $C_1$—$C_6$ à chaîne droite ou ramifiée et

Ar représente thiényle, furanyle, pyridyle, phényle ou phényle substitué par un ou plusieurs substituants choisis parmi un halogène et un alcoyle $C_1$—$C_6$;

leurs dérivés 2,3-dihydro; 5,6,7,8-tétrahydro et 2,3,5,6,7,8-hexahydro et les sels acceptables en pharmacie de tels composés.

2. Composés de formule I où B est CH,

$R_2$ représente —$CH_3$ ou —$CH_2CH_3$;

$R_5$ représente de l'hydrogène ou —$CH_3$;

$R_3$ représente —$NH_2$, —$NHC_2H_5$, —$CH_2CN$ ou —$CH_3$ et

$R_4$, étant attaché à la position 5-, 6- ou 7- du noyau, représente —O—$R_8$—Ar, —NH—$R_8$—Ar, —$R_8$—Ar, —CH=CH—Ar ou —CH=CH—$CH_2$—Ar, avec $R_8$ étant méthylène, éthylène ou propylène et Ar étant phényle, o-fluorophényle, p-fluorophényle, p-chlorophényle, thiényle ou furanyle.

3. Composés de formule I où B, $R_2$ et $R_5$ sont tels que définis à la revendication 2; $R_4$, étant attaché à la position 8 du noyau, est tel que défini à la revendication 2 et $R_3$ représente —$NH_2$ ou —$NHC_2H_5$.

4. Composés de formule I où B, $R_2$ et $R_5$ sont tels que définis à la revendication 2, $R_3$ est —$CH_2CN$ ou —$CH_3$ et $R_4$, étant attaché à la position 8 du noyau, est —CH=CH—Ar ou —CH=CH—$CH_2$—Ar, Ar étant tel que défini à la revendication 2.

5. Composés de formule

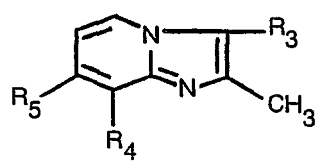

IA

où

$R_3$ est —$NH_2$;
$R_4$ est —$CH_2$—$CH_2$—Ar ou —$CH_2$—$CH_2$—$CH_2$—Ar et
$R_5$ est hydrogène ou méthyle, et
Ar est phényle ou 3-thiényle.

6. Composés de formule IA indiquée à la revendication 5, où
$R_3$ est —$NH_2$, —$CH_2CN$ ou —$CH_3$
$R_4$ est —CH=CH—Ar ou —CH=CH—$CH_2$—Ar et
$R_5$ est hydrogène ou méthyle, et
Ar est phényle ou 3-thiényle.

7. Composés de formule

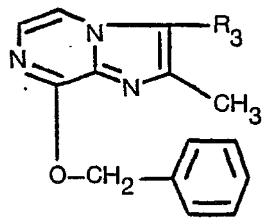

où

$R_3$ est alcoyle $C_1$—$C_6$, —$CH_2CN$, —$NH_2$, ou —$NHC_2H_5$.

8. Composé selon la revendication 5, étant la 3 - amino - 2 - méthyl - 8 - (2 - phényléthyl) - imidazo-[1,2 - a]pyridine.

9. Composés selon la revendication 6, étant la 2,3 - diméthyl - 8 - (2 - phényl - éthényl) - imidazo-[1,2 - a]pyridine et la 3 - cyanométhyl - 2 - méthyl - 8 - [E - 1 - (3 - phényl - propén - 1 - yl)] - imidazo-[1,2 - a]pyridine.

10. Composés selon la revendication 7, étant la 8 - benzyloxy - 3 - cyanométhyl - 2 - méthyl - imidazo[1,2 - a]pyrazine; la 8 - benzyloxy - 2,3 - diméthyl - imidazo[1,2 - a]pyrazine; et la 3 - amino - 8 - benzyloxy - 2 - méthyl - imidazo[1,2 - a]pyrazine.

11. Compositions pharmaceutiques comprenant un composé tel que défini selon l'une des revendications 1 à 10.

12. Procédé pour la préparation d'une composition pharmaceutique telle que définie à la revendication 11, caractérisé en ce qu'un composé tel que défini selon l'une des revendications 1 à 10 est mélangé à un ou plusieurs véhicules pharmaceutiques appropriés.

13. Procédé pour la préparation de composés tels que définis selon l'une des revendications 1 à 10, caractérisé en ce que soit

A: on fait réagir un composé de formule générale

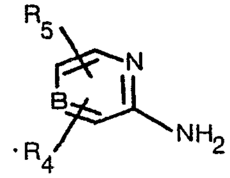

II

avec un composé de formule générale

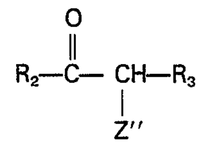 III

et dans les formules, $R_2$, $R_3$, $R_4$ et $R_5$ sont tels que définis à la revendication 1, à l'exception que tout groupe amino ou hydroxy libre présent dans $R_2$ et $R_3$ peut être protégé par un groupe protecteur qui est subséquemment retiré et Z'' représente un bon groupe partant; ou

B: pour la préparation de composés où $R_4$ est $-O-R_8-Ar$, $-O-CH_2-CH=CH_2$, $-NH-R_8-Ar$, $-R_8-Ar$, avec $R_8$ et Ar étant tels que définis à la revendication 1, on fait réagir un composé de formule générale

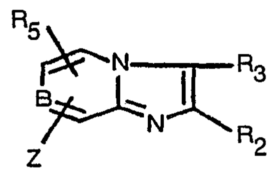 IV

avec un composé de formule générale

Hal—Z' V

et dans les formules ci-dessus, $R_2$, $R_3$ et $R_5$ sont tels que définis à la revendication 1, Hal représente Br, Cl ou I,

Z représente un halogène, OH ou $NH_2$ et

Z' représente $-R_8-Ar$ ou $CH_2-CH=CH_2$.

C: pour la préparation de composés où $R_4$ représente $-CH=CH-Ar$ ou $-CH=CH-CH_2-Ar$, un composé de formule IV où $R_2$, $R_3$ et $R_5$ sont tels que définis à la revendication 1 et Z représente CHO est soumis à une réaction de Wittig avec le réactif approprié de Wittig; ou

D: pour la préparation de composés de formule I où $R_4$ représente $-CH=CH-Ar$, la réaction d'un composé de formule IV où $R_2$, $R_3$ et $R_5$ sont tels que définis à la revendication 1 et Z représente un groupe phosphinylméthyle, avec un composé de formule Ar-CHO dans lequel Ar est tel que défini ci-dessus; ou

E: pour la préparation d'un composé de formule I où $R_2$, $R_4$ et $R_5$ sont tels que définis à la revendication 1 et $R_3$ représente $CH_2CN$, la réaction d'un composé de formule I où $R_3$ est un groupe $-CH_2X$, X étant un bon groupe partant, avec un cyanure de métal; ou

F: pour la préparation de composés de formule I où $R_2$, $R_3$, $R_4$ et $R_5$ sont tels que définis à la revendication 1, à l'exception qu'au moins l'un de $R_2$ et $R_3$ doit être un hydroxyalcoyle $C_1-C_6$, la réduction d'un composé de formule I où $R_2$ et/ou $R_3$ est un groupe $-(R')_nCOOR$, R' étant un groupe alcoylène $C_1-C_6$ ayant 1 à 5 atomes de carbone, n étant zéro ou un et R étant un groupe hydrocarbure; ou

G: pour la préparation de composés de formule I où $R_2$, $R_4$ et $R_5$ sont tels que définis à la revendication 1 et $R_3$ est —NO, la soumission d'un composé de formule I où $R_3$ est de l'hydrogène à une nitrosation, à la position 3, ou

H: pour la préparation de composés de formule I où $R_3$ est $-NH_2$ et $R_2$, $R_4$ et $R_5$ sont tels que définis à la revendication 1, la réduction d'un composé de formule I où $R_3$ est —NO ou $NO_2$; ou

I: pour la préparation de composés de formule I où $R_2$, $R_4$ et $R_5$ sont tels que définis à la revendication 1 et $R_3$ est $-NR_6R_7$ avec $R_6$ et/ou $R_7$ étant un alcoyle $C_1-C_6$, la soumission d'un composé de formule I où $R_3$ est $-NH_2$ à une alcoylation; ou

J: pour la préparation d'un composé de formule I où $R_2$, $R_4$ et $R_5$ sont tels que définis à la revendication 1 et $R_3$ est $-CH_2NC$, la soumission d'un composé où $R_3$ est un groupe

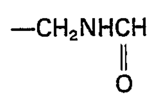

à une réaction avec $PCl_3$ en présence d'une amine;

en ce qu'un composé ainsi obtenu de formule I, si on le souhaite, est réduit en dérivé 2,3-dihydro-5,6,7,8-tétrahydro- ou 2,3,5,6,7,8-hexahydro correspondant; et en ce qu'un composé obtenu selon l'un des procédés décrits ci-dessus, si on le souhaite, est transformé en un sel.

14. Procédé selon la revendication 13, caractérisé en ce que dans le procédé

A: Z'' représente un halogène, du tosyle ou du mésyle et la réaction est effectuée par chauffage des réactifs ensemble dans un solvant inerte;

B: quand Z représente un halogène, on utilise un catalyseur de cuivre;

C: le réactif de Wittig utilisé a pour formule

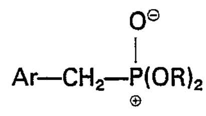

avec Ar étant tel que défini à la revendication 1, R étant un groupe hydrocarbure;

E: X représente un halogène, un alcoxy, un aryloxy, un mésyle, un tosyle, un groupe quaternaire, de préférence $\overset{\oplus}{N}(CH_3)_3I^{\ominus}$, ou un groupe quaternaire où l'ion quaternaire est un contre-ion non nucléophile choisi parmi $BF_4^{\ominus}$, $PF_6^{\ominus}$, $CF_3SO_3^{\ominus}$, et $FSO_3^{\ominus}$ et le cyanure de métal est un cyanure d'un métal alcalin;

F: la réduction est effectuée au moyen d'hydrure de lithium aluminium;

G: la nitrosation est effectuée au moyen d'un nitrite, de préférence du nitrite de sodium, en présence de HCl concentré;

H: la réduction est effectuée au moyen de poudre de zinc dans l'acide acétique.

**Revendications pour l'Etat contractant: AT**

1. Procédé pour la préparation d'imidazo[1,2-a]pyridines et pyrazines de formule générale

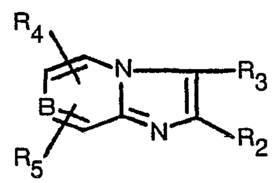

I

où B est CH ou N, et quand B est CH, alors

$R_2$ est hydrogène, alcoyle $C_1$—$C_6$ ou hydroxyalcoyle $C_1$—$C_6$;

$R_5$ est hydrogène, halogène ou alcoyle $C_1$—$C_6$;

et *soit*

$R_3$ est alcoyle $C_1$—$C_6$, —$CH_2CN$, hydroxyalcoyle $C_1$—$C_6$, —NO, —$CH_2NC$,

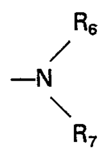

ou, à condition que $R_2$ ne soit pas de l'hydrogène, également de l'hydrogène et

$R_4$, étant attaché à toute position 5-, 6- ou 7- du noyau, représente l'un des groupements —O—$R_8$—Ar, —NH—$R_8$—Ar, —$R_8$—Ar, —CH=CH—Ar, —CH=CH—$CH_2$—Ar ou —O—$CH_2$—CH=$CH_2$; ou

$R_3$ est tel que défini ci-dessus et

$R_4$, étant attaché à la position 8 du noyau, représente l'un des groupements: —CH=CH—Ar, —CH=CH—$CH_2$—Ar ou —O—$CH_2$—CH=$CH_2$; ou

$R_3$ est —NO, —$CH_2NC$ ou

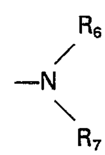

et

$R_4$, étant attaché à la position 8 du noyau, représente —O—$R_8$—Ar, —NH—$R_8$—Ar ou —$R_8$—Ar;

et quand B est N, alors

$R_5$ est tel que défini ci-dessus,

$R_2$ et $R_3$ sont indépendamment choisis parmi l'hydrogène, un alcoyle $C_1$—$C_6$, un hydroxyalcoyle $C_1$—$C_6$, —$CH_2CN$, —NO et —$NR_6R_7$ et

$R_4$ est —O—$R_8$—Ar, —NH—$R_8$—Ar, —$R_8$—Ar, —CH=CH—Ar ou —CH=CH—$CH_2$—Ar;

et dans les définitions ci-dessus, $R_6$ et $R_7$ sont indépendamment choisis parmi l'hydrogène ou un alcoyle $C_1$—$C_6$;

$R_8$ est un groupe alcoylène $C_1$—$C_6$ à chaîne droite ou ramifiée et

Ar représente thiényle, furanyle, pyridyle, phényle ou phényle substitué par un ou plusieurs substituants choisis parmi un halogène et un alcoyle $C_1$—$C_6$;
leurs dérivés 2,3-dihydro; 5,6,7,8-tétrahydro et 2,3,5,6,7,8-hexahydro et les sels acceptables en pharmacie de tels composés; caractérisé en ce que

A: on fait réagir un composé de formule générale

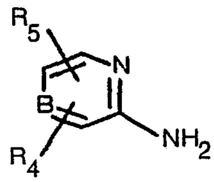

II

avec un composé de formule générale

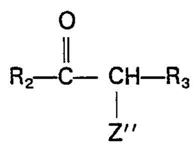

III

et dans les formules, $R_2$, $R_3$, $R_4$ et $R_5$ sont tels que définis à la revendication 1, à l'exception que tout groupe amino ou hydroxy libre présent dans $R_2$ ou $R_3$ peut être protégé par un groupe protecteur qui est subséquemment retiré et Z'' représente un bon groupe partant; ou

B: pour la préparation de composés où $R_4$ est —O—$R_8$—Ar, —O—$CH_2$—CH=$CH_2$, —NH—$R_8$—Ar, —$R_8$—Ar, avec $R_8$ et Ar étant tels que définis à la revendication 1, on fait réagir un composé de formule générale

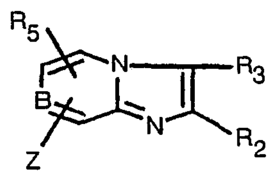

IV

avec un composé de formule générale

Hal—Z'

et dans les formules ci-dessus, $R_2$, $R_3$ et $R_5$ sont tels que définis à la revendication 1, Hal représente Br, Cl ou I,

Z représente un halogène, OH ou $NH_2$ et

Z' représente —$R_8$—Ar ou $CH_2$—CH=$CH_2$.

C: pour la préparation de composés où $R_4$ représente —CH=CH—Ar ou —CH=CH—$CH_2$—Ar, un composé de formule IV où $R_2$, $R_3$ et $R_5$ sont tels que définis à la revendication 1 et Z représente CHO est soumis à une réaction de Wittig avec le réactif approprié de Wittig; ou

D: pour la préparation de composés de formule I où $R_4$ représente —CH=CH—Ar, la réaction d'un composé de formule IV où $R_2$, $R_3$ et $R_5$ sont tels que définis à la revendication 1 et Z représente un groupe phosphinylméthyle, avec un composé de formule Ar-CHO par lequel Ar est tel que défini ci-dessus; ou

E: pour la préparation d'un composé de formule I où $R_2$, $R_5$ et $R_5$ sont tels que définis à la revendication 1 et $R_3$ représente $CH_2CN$, la réaction d'un composé de formule I où $R_3$ est un groupe —$CH_2X$, X étant un bon groupe partant, avec un cyanure de métal; ou

F: pour la préparation de composés de formule I où $R_2$, $R_3$, $R_4$ et $R_5$ sont tels que définis à la revendication 1, à l'exception qu'au moins l'un de $R_2$ et $R_3$ doit être un hydroxyalcoyle inferieur, la réduction d'un composé de formule I où $R_2$ et/ou $R_3$ est un groupe —$(R')_n$COOR, R' étant un groupe alcoylène inferieur ayant 1 à 5 atomes de carbone, n étant zéro ou un et R étant un groupe hydrocarbure; ou

G: pour la préparation de composés de formule I où $R_2$, $R_4$ et $R_5$ sont tels que définis à la revendication 1, et $R_3$ est —NO, la soumission d'un composé de formule I où $R_3$ est de l'hydrogène à une nitrosation, à la position 3, ou

H: pour la préparation de composés de formule I où $R_3$ est —$NH_2$ et $R_2$, $R_4$ et $R_5$ sont tels que définis à la revendication 1, la réduction d'un composé de formule I où $R_3$ est —NO ou $NO_2$; ou

I: pour la préparation de composés de formule I où $R_2$, $R_4$ et $R_5$ sont tels que définis à la revendication 1 et $R_3$ est —$NR_6R_7$ avec $R_6$ et/ou $R_7$ étant un alcoyle inferieur, la soumission d'un composé de formule I où $R_3$ est —$NH_2$ à une alcoylation; ou

J: pour la préparation d'un composé de formule I où $R_2$, $R_4$ et $R_5$ sont tels que définis à la revendication 1 et $R_3$ est $-CH_2NC$, la soumission d'un composé où $R_3$ est un groupe

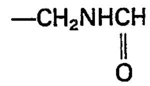

à une réaction avec $PCl_3$ en présence d'une amine;

en ce qu'un composé ainsi obtenu de formule I, si on le souhaite, est réduit en dérivé 2,3-dihydro-, 4,5,6,7,8-tétrahydro- ou 2,3,5,6,7,8-hexahydro correspondant; et en ce qu'un composé obtenu selon l'un des procédés décrits ci-dessus, si on le souhaite, est transformé en un sel.

2. Procédé selon la revendication 1, caractérisé en ce que dans le procédé

A: Z'' représente un halogène, du tosyle ou du mésyle et la réaction est effectuée par chauffage des réactifs ensemble dans un solvant inerte;

B: quand Z représente un halogène, on utilise un catalyseur de cuivre;

C: le réactif de Wittig utilisé a pour formule

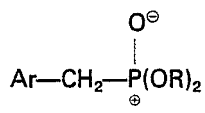

avec Ar étant tel que défini à la revendication 1, R étant un groupe hydrocarbure;

E: X représente un halogène, un alcoxy, un aryloxy, un mésyle, un tosyle, un groupe quaternaire, de préférence $\overset{\oplus}{N}(CH_3)_3I^{\ominus}$, ou un groupe quaternaire où l'ion quaternaire est un contre-ion non nucléophile choisi parmi $BF_4^{\ominus}$, $PF_6^{\ominus}$, $CF_3SO_3^{\ominus}$, et $FSO_3^{\ominus}$ et le cyanure de métal est un cyanure d'un métal alcalin;

F: la réduction est effectuée au moyen d'hydrure de lithium aluminium;

G: la nitrosation est effectuée au moyen d'un nitrite, de préférence du nitrite de sodium en présence de HCl concentré;

H: la réduction est effectuée au moyen de poudre de zinc dans l'acide acétique.

3. Procédé selon la revendication 1 ou 2, caractérisé en ce que les composés de formule I où B est CH,

$R_2$ représente $-CH_3$ ou $-CH_2CH_3$;

$R_5$ représente de l'hydrogène ou $-CH_3$;

$R_3$ représente $-NH_2$, $-NHC_2H_5$, $-CH_2CN$ ou $-CH_3$ et

$R_4$, étant attaché à la position 5-, 6- ou 7- du noyau, représente $-O-R_8-Ar$, $-NH-R_8-Ar$, $-R_8-Ar$, $-CH{=}CH-Ar$ ou $-CH{=}CH-CH_2-Ar$, avec $R_8$ étant méthylène, éthylène ou propylène et Ar étant phényle, o-fluorophényle, p-fluorophényle, p-chlorophényle, thiényle ou furanyle, sont préparés.

4. Procédé selon la revendication 1 ou 2, caractérisé en ce que les composés de formule I où B, $R_2$ et $R_5$ sont tels que définis à la revendication 3; $R_4$ étant attaché à la position 8 du noyau, est tel que défini à la revendication 3 et $R_3$ représente $-NH_2$ ou $-NHC_2H_5$, sont préparés.

5. Procédé selon la revendication 1 ou 2, caractérisé en ce que les composés de formule I où B, $R_2$ et $R_5$ sont tels que définis à la revendication 3, $R_3$ est $-CHCN$ ou $-CH_3$ et $R_4$, étant attaché à la position 8 du noyau, est $-CH{=}CH-Ar$ ou $-CH{=}CH-CH_2-Ar$, avec Ar étant tel que défini à la revendication 3, sont préparés.

6. Procédé selon la revendication 1 ou 2, caractérisé en ce que les composés de formule

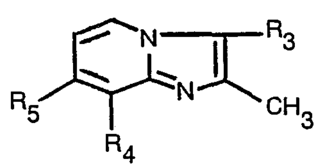

IA

où

$R_3$ est $-NH_2$;

$R_4$ est $-CH_2-CH_2-Ar$ ou $-CH_2-CH_2-CH_2-Ar$ et

$R_5$ est hydrogène ou méthyle, et

Ar est phényle ou thiényle, sont préparés.

7. Procédé selon la revendication 1 ou 2, caractérise en ce que les composés de formule IA indiqués à la revendication 6, où

$R_3$ est $-NH_2$, $-CH_2CN$ ou $-CH_3$

$R_4$ est $-CH{=}CH-Ar$ ou $-CH{=}CH-CH_2-Ar$ et

$R_5$ est hydrogène ou méthyle, et

Ar est phényle ou 3-thiényle, sont préparés.

8. Procédé selon la revendication 1 ou 2, caractérisé en ce que les composés de formule

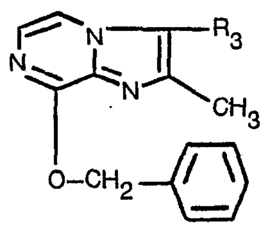

où

$R_3$ est alcoyle $C_1$—$C_6$, —$CH_2CN$, —$NH_2$, ou —$NHC_2H_5$, sont préparés.

9. Procédé selon la revendication 1 ou 2, caractérisé en ce que les composés 3 - amino - 2 - méthyl - 8 - (2 - phényléthyl) - imidazo[1,2 - a]pyridine; 2,3 - diméthyl - 8 - (2 - phényl - éthényl) - imidazo[1,2 - a]pyridine et 3 - cyanométhyl - 2 - méthyl - 8 - [E - 1 - (3 - phényl - propén - 1 -yl)] - imidazo[1,2 - a]pyridine, sont préparés.

10. Procédé selon la revendication 1 ou 2, caractérisé en ce que les composés 8 - benzyloxy - 3 - cyanométhyl - 2 - méthyl - imidazo[1,2 - a]pyrazine; 8 - benzyloxy - 2,3 - diméthyl - imidazo[1,2 - a]-pyrazine et 3 - amino - 8 - benzyloxy - 2 - méthyl - imidazo[1,2 - a]pyrazine sont préparés.